# EUROPEAN PATENT APPLICATION

(11) **EP 2 151 431 A1**
(43) Date of publication of application: **10.02.2010**
(21) Application number: 08752105.0
(22) Date of filing: 25.04.2008
(51) Int. Cl.: C07C 235/46, A01N 37/40, A01P 3/00, C07C 235/54

(54) **AMIDE COMPOUND AND USE THEREOF**

(30) Priority: 27.04.2007 JP 2007118646
(71) Applicant: Sumitomo Chemical Company, Limited, Tokyo 104-8260 (JP)
(72) Inventor: KOMORI, Takashi, Nerima-ku Tokyo 176-0013 (JP); KUBOTA, Mayumi, Toyonaka-shi Osaka 561-0802 (JP); MATSUZAKI, Yuichi, Toyonaka-shi Osaka 560-0021 (JP)
(74) Representative: Vossius & Partner
(86) International application number: PCT/JP2008/058031
(87) International publication number: WO 2008/136387

(57) **Abstract**

Disclosed is an amide compound represented by the formula (1) below, which has excellent plant disease controlling activity. 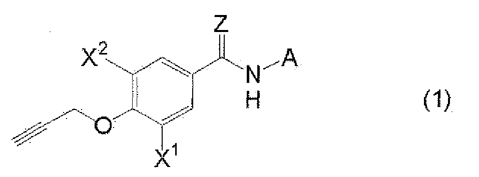 In the formula, X¹ represents a fluorine atom or a methoxy group; X² represents a chlorine atom, a bromine atom, an iodine atom, a C₁-C₄ alkyl group or the like; Z represents an oxygen atom or a sulfur atom; and A represents an A¹-CR¹¹R¹²R¹³ group or A²-Cy group, wherein A¹ and A² each represents a single bond or the like, R¹¹ and R¹² independently represent a C₁-C₄ alkyl group, R¹³ represents H, a C₁-C₄ alkyl group or the like, and Cy represents a C₃-C₆ cycloalkyl group or the like.

## Description

### Technical Field

The present invention relates to an amide compound and use thereof.

### Background Art

Drugs for controlling plant diseases have been conventionally developed. Compounds having plant disease controlling activity have been found out and have been put into practice.

### Disclosure of Invention

### Problems to be solved by the invention

An object of the present invention is to provide a compound having excellent plant disease controlling activity.

### Means for solving the problems

The present inventors studied in order to find out a compound having excellent plant disease controlling efficacy and, as a result, found that an amide compound represented by the following formula (1) has excellent plant disease controlling activity. Thus the present invention was completed.
The present invention provides an amide compound represented by the formula (1): 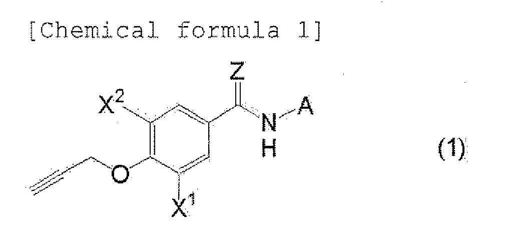 wherein,
X¹ represents a fluorine atom or a methoxy group,
X² represents a chlorine atom, a bromine atom, an iodine atom, a C₁-C₄ alkyl group, a C₂-C₄ alkenyl group, a C₂-C₄ alkynyl group, a C₁-C₄ haloalkyl group, a C₂-C₄ alkoxy group, a C₁-C₄ alkylthio group, a hydroxy C₁-C₄ alkly group, a nitro group, a cyano group, a formyl group, a NR¹R² group, a CO₂R³ group, a CONR⁴R⁵ group, or a phenyl group optionally substituted with at least one group selected from the group consisting of a methyl group, a halogen atom, a cyano group and a nitro group,
Z represents an oxygen atom or a sulfur atom,
A represents an A¹-CR¹¹R¹²R¹³ group or A²-Cy group,
A¹ represents a single bond or a CH₂ group,
A² represents a single bond, a CH₂ group, a CH(CH₃) group, a C(CH₃)₂ group or a CH(CH₂CH₃) group,
Cy represents a C₃-C₆ cycloalkyl group optionally substituted with at least one group selected from the group consisting of a C₁-C₄ alkyl group, a C₂-C₄ alkenyl group, a C₂-C₆ alkynyl group, a halogen atom, a hydroxyl group, a cyano group, a carboxyl group and a C₂-C₅ alkoxycarbonyl group,
R¹ and R² independently represent a hydrogen atom, a C₁-C₄ alkyl group, a C₃-C₄ alkenyl group, a C₃-C₄ alkynyl group, a C₂-C₄ haloalkyl group, a C₂-C₅ alkylcarbonyl group, a C₂-C₅ alkoxycarbonyl group or a C₁-C₄ alkylsulfonyl group,
R³ represents a C₁-C₄ alkyl group, a C₃-C₄ alkenyl group or a C₃-C₄ alkynyl group,
R⁴ represents a hydrogen atom, a C₁-C₄ alkyl group, a C₃-C₄ alkenyl group, a C₃-C₄ alkynyl group, a C₂-C₄ haloalkyl group, a C₂-C₅ alkylcarbonyl group, a C₂-C₅ alkoxycarbonyl group, or a C₁-C₄ alkylsulfonyl group,
R⁵ represents a hydrogen atom, a C₁-C₄ alkyl group, a C₃-C₄ alkenyl group, a C₃-C₄ alkynyl group or a C₂-C₄ haloalkyl group,
R¹¹ and R¹² independently represent a C₁-C₄ alkyl group, and
R¹³ represents a hydrogen atom, a C₁-C₄ alkyl group, a C₂-C₄ alkenyl group, a C₂-C₄ alkynyl group, a cyano group, a carboxyl group or a C₂-C₅ alkoxycarbonyl group (hereinafter, referred to as the compound of the present invention), a plant disease controlling agent containing the compound of the present invention as an active ingredient, and a plant disease controlling method which comprises treating a plant or soil with an effective amount of the compound of the present invention.

### Effect of the invention

The compound of the present invention has excellent plant disease controlling activity and therefore, it is useful as an active ingredient of a plant disease controlling agent.

### Best Mode for Carrying Out the Invention

In the present invention, examples of the C₁-C₄ alkyl group represented by X² include a methyl group, an ethyl group, a 1-methylethyl group, a 1,1-dimethylethyl group, a propyl group and a 1-methylpropyl group.
Examples of the C₂-C₄ alkenyl group represented by X² include a vinyl group, a 1-propenyl group, a 2-propenyl group, a 2-butenyl group and a 3-butenyl group.
Examples of the C₂-C₄ alkynyl group represented by X² include an ethynyl group, a 1-propynyl group, a 2-propynyl group, a 3-butynyl group.
Examples of the C₁-C₄ haloalkyl group represented by X² include a fluoromethyl group, a chloromethyl group, a bromomethyl group, a difluoromethyl group, a dichloromethyl group, a dibromomethyl group, a trifluoromethyl group, a trichloromethyl group, a dichlorofluoromethyl group, a chlorodifluoromethyl group, a 1,1-difluoroethyl group, a 2,2,2-trifluoroethyl group, a 2-fluoroethyl group, a 3-fluoropropyl group, a 4-fluorobutyl group and a 1-chloroethyl group.
Examples of the C₂-C₄ alkoxy group represented by X² include an ethoxy group, a 1-methylethoxy group, a 1,1-dimethylethoxy group, a propoxy group, a 1-methylpropoxy group, a 2-methylpropoxy group and a butoxy group.
Examples of the C₁-C₄ alkylthio group represented by X² include a methylthio group, an ethylthio group, a 1-methylethylthio group, a 1,1-dimethylethylthio group, a propylthio group and a 1-methylpropylthio group.
Examples of the hydroxyl C₁-C₄ alkyl group represented by X² include a hydroxymethyl group, a 1-hydroxyethyl group and a 2-hydroxyethyl group.
Examples of the phenyl group optionally substituted with at least one group selected from the group consisting of a methyl group, a halogen atom, a cyano group and a nitro group which is represented by X² include a phenyl group, a 2-methylphenyl group, a 3-methylphenyl group, a 4-methylphenyl group, a 2-fluorophenyl group, a 3-fluorophenyl group, a 4-fluorophenyl group, a 2-chlorophenyl group, a 3-chlorophenyl group, a 4-chlorophenyl group, a 4-cyanophenyl group and a 4-nitrophenyl group.

Examples of the C₁-C₄ alkyl group represented by R¹ include a methyl group, an ethyl group, a 1-methylethyl group, a 1,1-dimethylethyl group, a propyl group and a 1-methylpropyl group. Examples of the C₃-C₄ alkenyl group represented by R¹ include a 1-propenyl group, a 2-propenyl group, a 2-butenyl group and a 3-butenyl group.
Examples of the C₃-C₄ alkynyl group represented by R¹ include a 1-propynyl group, a 2-propynyl group and a 3-butynyl group.
Examples of the C₂-C₄ haloalkyl group represented by R¹ include a 1,1-difluoroethyl group, a 2,2,2-trifluoroethyl group, a 2-fluoroethyl group, a 3-fluoropropyl group, a 4-fluorobutyl group and a 1-chloroethyl group.
Examples of the C₂-C₅ alkylcarbonyl group represented by R¹ include an acetyl group, an ethylcarbonyl group, a 1-methylethyloarboryl group and a 1,1-dimethylethylcarbonyl group.
Examples of the C₂-C₅ alkoxycarbonyl group represented by R¹ include a methoxycarbonyl group, an ethoxycarbonyl group, a 1-methylethoxycarbonyl group and a 1,1-dimethylethoxycarbonyl group.
Examples of the C₁-C₄ alkylsulfonyl group represented by R¹ include a methylsulfonyl group, an ethylsulfonyl group, a 1-methylethylsulfonyl group and a 1,1-dimethylethylsulfonyl group.
Examples of the C₁-C₄ alkyl group represented by R² include a methyl group, an ethyl group, a 1-methylethyl group, a 1,1-dimethylethyl group, a propyl group and a 1-methylpropyl group.
Examples of the C₃-C₄ alkenyl group represented by R² include a 1-propenyl group, a 2-propenyl group, a 2-butenyl group and a 3-butenyl group.
Examples of the C₃-C₄ alkynyl group represented by R² include a 1-propynyl group, a 2-propynyl group and a 3-butynyl group.
Examples of the C₂-C₄ haloalkyl group represented by R² include a 1,1-difluoroethyl group, a 2,2,2-trifluoroethyl group, a 2-fluoroethyl group, a 3-fluoropropyl group, a 4-fluorobutyl group and a 1-chloroethyl group.
Examples of the C₂-C₅ alkylcarbonyl group represented by R² include an acetyl group, an ethylcarbonyl group, a 1-methylethylcarbonyl group and a 1,1-dimethylethylcarbonyl group.
Examples of the C₂-C₅ alkoxycarbonyl group represented by R² include a methoxycarbonyl group, an ethoxycarbonyl group, a 1-methylethoxycarbonyl group and a 1,1-dimethylethoxycarbonyl group.
Examples of the C₁-C₄ alkylsulfonyl group represented by R² include a methylsulfonyl group, an ethylsulfonyl group, a 1-methylethylsulfonyl group and a 1,1-dimethylethylsulfonyl group.
Examples of the C₁-C₄ alkyl group represented by R³ include a methyl group, an ethyl group, a 1-methylethyl group, a 1,1-dimethylethyl group, a propyl group, and a 1-methylpropyl group. Examples of the C₃-C₄ alkenyl group represented by R³ include a 1-propenyl group, a 2-propenyl group, a 2-butenyl group and a 3-butenyl group.
Examples of the C₃-C₄ alkynyl group represented by R³ include a 1-propynyl group, a 2-propynyl group, and a 3-butynyl group.
Examples of the C₁-C₄ alkyl group represented by R⁴ include a methyl group, an ethyl group, a 1-methylethyl group, a 1,1-dimethylethyl group, a propyl group and a 1-methylpropyl group.
Examples of the C₃-C₄ alkenyl group represented by R⁴ include a 1-propenyl group, a 2-propenyl group, a 2-butenyl group and a 3-butenyl group.
Examples of the C₃-C₄ alkynyl group represented by R⁴ include a 1-propynyl group, a 2-propynyl group, and a 3-butynyl group.
Examples of the C₂-C₄ haloalkyl group represented by R⁴ include a 1,1-difluoroethyl group, a 2,2,2-trifluroethyl group, a 2-fluoroethyl group, a 3-fluoropropyl group, a 4-fluorobutyl group, and a 1-chloroethyl group.
Examples of the C₂-C₅ alkylcarbonyl group represented by P⁴ include an acetyl group, an ethylcarbonyl group, a 1-methylethylcarbonyl group and a 1,1-dimethylethylcarbonyl group.
Examples of the C₂-C₅ alkoxycarbonyl group represented by R⁴ include a methoxycarbonyl group, an ethoxycarbornyl group, a 1-methylethoxycarbonyl group and a 1,1-dimethylethoxycarbonyl group.
Examples of the C₁-C₄ alkylsulfonyl group represented by R⁴ include a methylsulfonyl group, an ethylsulfonyl group, a 1-methylethylsulfonyl group and a 1,1-dimethylethylsulfonyl group.
Examples of the C₁-C₄ alkyl group represented by R₅ include a methyl group, an ethyl group, a 1-methylethyl group, a 1,1-dimethylethyl group, a propyl group, and a 1-methylpropyl group.
Examples of the C₃-C₄ alkenyl group represented by R₅ include a 1-propenyl group, a 2-propenyl group, a 2-butenyl group and a 3-butenyl group.
Examples of the C₃-C₄ alkynyl group represented by R₅ include a 1-propynyl group, a 2-propynyl group and a 3-butynyl group.
Examples of the C₂-C₄ haloalkyl group represented by R₅ include a 1,1-difluoroethyl group, a 2,2,2-trifluoroethyl group, a 2-fluoroethyl group, a 3-fluoropropyl group, a 4-fluorobutyl group and a 1-chloroethyl group.

Examples of the NR¹R² group include an amino group, a methylamino group, a dimethylamino group, an ethylamino group, a 2-propenylamino group, a propynylamino group, a 2-chloroethylamino group, an acetylamino group, a propionylamino group, a 1,1-dimethylethylcarbonylamino group, a methoxycarbonylamino group, an ethoxycarbonylamino group, a methanesulfonylamino group, an N-acetyl-N-methylamino group, an N-ethoxycarbonyl-N-methylamino group and an N-methanesulfonyl-N-methylamino group.
Examples of the CONR⁴R⁵ group include a carbamoyl group, a methylcarbamoyl group, a dimethylcarbamoyl group, an ethylmethylcarbamoyl group, a (2-propenyl)carbamoyl group, a (2-propynyl)carbamoyl group and a 2-chloroethylcarbamoyl group.

Examples of the C₁-C₄ alkyl group represented by R¹¹ include a methyl group, an ethyl group, a 1-methylethyl group, a 1,1-dimethylethyl group, a propyl group, and a 1-methylpropyl group.
Examples of the C₁-C₄ alkyl group represented by R¹² include a methyl group, an ethyl group, a 1-methylethyl group, a 1,1-dimethylethyl group, a propyl group, and a 1-methylpropyl group.
Examples of the C₁-C₄ alkyl group represented by R¹³ include a methyl group, an ethyl group, a 1-methylethyl group, a 1,1-dimethylethyl group, a propyl group, and a 1-methylpropyl group.
Examples of the C₂-C₄ alkenyl group represented by R¹³ include a vinyl group, a 1-propenyl group, a 2-propenyl group, a 2-butenyl group and a 3-butenyl group.
Examples of the C₂-C₄ alkynyl group represented by R¹³ include an ethynyl group, a 1-propynyl group, a 2-propynyl group and a 3-butynyl group.
Examples of the C₂-C₅ alkoxycarbonyl group represented by R¹³ include a methoxycarbonyl group, an ethoxycarbonyl group and a 1-methylethoxycarbonyl group.

Examples of the group represented by CR¹¹R¹²R¹³ include a 1-methylethyl group, a 1-methylpropyl group, a 1-methylbutyl group, a 1-methylpropyl group, a 1,1-diemethylethyl group, a 1,1-dimethylpropyl group, a 1,2-dimethylpropyl group, a 1,2-dimethylbutyl group, a 1,1,2-trimethylpropyl group, a 1,2,2-trimethylpropyl group, a 1,1,2,2-tetramethylpropyl group and a 1-cyano-1,2-dimethylpropyl group.
In the C₃-C₆ cycloalkyl group optionally substituted with at least one group selected from the group consisting of a C₁-C₄ alkyl group, a C₂-C₄ alkenyl group, a C₂-C₄ alkynyl group, a halogen atom, a hydroxyl group, a cyano group, a carboxyl group and a C₂-C₅ alkoxycarbonyl group which is represented by Cy;
examples of the C₁-C₄ alkyl group as a substituent include a methyl group, an ethyl group, a propyl group and an isopropyl group,
examples of the C₂-C₄ alkenyl group as a substituent include a vinyl group, a 1-propenyl group, a 2-propenyl group, a 2-butenyl group and a 3-butenyl group,
examples of the C₂-C₄ alkynyl group as a substituent include an ethynyl group, a 1-propynyl group, a 2-propynyl group and a 3-butynyl group,
examples of the halogen atom as a substituent include a fluorine atom, a chlorine atom, a bromine atom, and an iodine atom, and
examples of the C₂-C₅ alkoxycarbonyl group as a substituent include a methoxycarbonyl group and an ethoxycarbonyl group.
Examples of the C₃-C₆ cycloalkyl in the C₃-C₆ cycloalkyl group optionally substitute with at least one group selected from the group consisting of a C₁-C₄ alkyl group, a C₂-C₄ alkenyl group, a C₂-C₄ alkynyl group, a chlorine atom, a hydroxyl group, a cyano group, a carboxyl group and a C₂-C₅ alkoxycarbonyl group which is represented by Cy include a cyclopropyl group, a cyclobutyl group, a cyclopentyl group, and a cyclohexyl group.

Examples of the group represented by A²-Cy specifically include the following groups:
a cyclopropyl group, a 2,2,3,3-tetramethylcyclopropyl group;
a cyclobutyl group;
a cyclopentyl group, a 2-methylcyclopentyl group, a 2-hydroxycyclopentyl group, a 2-chlorocyclopentyl group, a 2-bromocyclopentyl group, a 2,2-dimethylcyclopentyl group, a 2-fluoro-2-methylcyclopentyl group, a 2-chloro-2-methylcyclopentyl group, a 2-hydroxy-2-methylcyclopentyl group, a 2,2-difluorocyclopentyl group, a 3-methylcyclopentyl group;
a cyclohexyl group, a 1-methylcyclohexyl group, a 2-methylcyclohexyl group, a 3-methylcyclohexyl group, a 4-methylcyclohexyl group, a 2-trifluoromethylcyclohexyl group, a 2,2-dimethylcyclohexyl group, a 2-fluoro-2-methylcyclohexyl group, a 2-chloro-2-Tnethylcyclohexyl group, a 2-hydroxy-2-methylcyclohexyl group, a 2,2-difluorocyclohexyl group, a 2,3-dimethylcyclohexyl group, a 2,6-dimethylcyclohexyl group, a 2-ethylcyclohexyl group, a 2-chlorocyclohexyl group, a 2-fluorocyclohexyl group, a 2-bromocyclohexyl group, a 2-iodocyclohexyl group, a 2-hydroxycyclohexyl group, a 1-cyanocyclohexyl group, a 2-cyanocyclohexyl group, a 1-carboxycyclohexyl group a 1-(methoxycarbonyl)cyclohexyl group, a 1-(ethoxycarbonyl)cyclohexyl group, a 2-(methoxycarbonyl)cyclohexyl group, a 2-(ethoxycarbonyl)cyclohexyl group;

a cyclopropylmethyl group, a (1-methylcyclopropyl)methyl group, a (2-methylcyclopropyl)methyl group, a (1-hydroxycyclopropyl)methyl group, a (2-hydroxycyclopropyl)methyl group, a 2,2,3,3-tetramethylcyclopropyl)methyl group, a (1-fluorocyclopropyl)methyl group, a (1-chlorocyclopropyl)methyl group, a (1-cyanocyclopropyl)methyl group, a (1-ethoxycarbonylcyclopropyl)methyl group, a (1-methoxycarbonylcyclopropyl)methyl group;
a cyclobutylmethyl group, a (1-methylcyclobutyl)methyl group, a (2-methylcyclobutyl)methyl group, a (3-methylcyclobutyl)methyl group, a (1-hydroxycyclobutyl)methyl group, a (2-hydroxycyclobutyl)methyl group, a (3-hydroxycyclobutyl)methyl group, a (1-fluorocyclobutyl)methyl group, a (1-chloracyclobutyl)methyl group, a (1-cyanocyclobutyl)methyl group, a (1-ethoxycarbonylcyclobutyl)methyl group, a (1-methoxycarbonylcyclobutyl)methyl group;
a cyclopentylmethyl group, a (1-methylcyclopentylmehyl) group, a (2-methylcyclopentyl)methyl group, a (3-methylcyclopentyl)methyl group, a (1-hydroxycyclopentyl)methyl group, a (2-hydroxycyclopentyl)methyl group, a (3-hydroxycyclopentyl)methyl group, a (1-fluorocyclopentyl)methyl group, a (1-chlorocyclopentyl)methyl group, a (1-cyanocyclopentyl)methyl group, a (1-ethoxycarbonylcyclopentyl)methyl group, a (1-methoxycarbonylcyclopenyl)methyl group;
a cyclohexylmethyl group, a (1-methylcyclohexyl)methyl group, a (2-methylcyclohexyl)methyl group, a (3-methylcyclohexyl)methyl group, a (4-methylcyclohexyl)methyl group, a (2,3-dimethylcyclohexyl)methyl group, a (1-hydroxycyclohexyl)methyl group, a (2-hydroxycyclohexyl)methyl group, a (3-hydroxycyclohexyl)methyl group, a (4-hydroxycyclohexyl)methyl group, a (1-fluorocyclohexyl)methyl group, a (1-chlorocyclohexyl)methyl group, a (1-cyanocyclohexyl)methyl group, a (1-ethoxycarbonylcyclohexyl)methyl group, a (1-methoxycarbonylcyclohexyl)methyl group, a (1-ethynylcyclohexyl)methyl group, a (2-fluorocyclohexyl)methyl group, a (2-chlorocyclohexyl)methyl group, a (2-cyanocyclohexyl)methyl group, a (2-ethoxycarbonylcyclohexyl)methyl group, a (2-methoxycarbonylcyclohexyl)methyl group;

a 1-(cyclopropyl)ethyl group, a 1-(1-methylcyclopropyl)ethyl group, a 1-(2-methylcyclopropyl)ethyl group, a 1-(1-hydroxycyclopropyl)ethyl group, a 1-(2-hydroxycyclopropyl)ethyl group, a 1-(2,2,3,3-tetramethylcyclopropyl)ethyl group, a 1-(1-fluorocyclopropyl)ethyl group, a 1-(1-chlorocyclopropyl)ethyl group, a 1-(1-cyanocyclopropyl)ethyl group, a 1-(1-ethoxycarbonylcylclopropyl)ethyl group, a 1-(1-methoxycarbonylcyclopropyl)ethyl group;
a 1-(cyclobutyl)ethyl group, a 1-(1-methylcyolobutyl)ethyl group, a 1-(2-methylcyclobutyl)ethyl group, a 1-(3-methylcyolobutyl)ethyl group,
a 1-(1-hydroxycyclobutyl)ethyl group, a 1-(2-hydroxycyclobutyl)ethyl group, a 1-(3-hydroxycyclobutyl)ethyl group, a 1-(1-fluorocyclobutyl)ethyl group, a 1-(l-chlorocyclobutyl)ethyl group, a 1-(1-cyanocyclobutyl)ethyl group, a 1-(1-ethoxycarbonylcyclobutyl)ethyl group, a 1-(1-methoxycarbonylcyclobutyl)ethyl group;
a 1-(cyclopentyl)ethyl group, a 1-(1-methylcyclopentyl)ethyl group, a 1-(2-methylcyclopentyl)ethyl group, a 1-(3-methylcyclopentyl)ethyl group, a 1-(1-hydroxycyclopentyl)ethyl group, a 1-(2-hydroxycyclopentyl)ethyl group, a 1-(3-hydroxycyclopentyl)ethyl group, a 1-(1-fluorocyclopentyl)ethyl group, a 1-(1-chlorocyclopentyl)ethyl group, a 1-(1-cyanocyclopentyl)ethyl group, a 1-(1-ethoxycarbonylcyclopentyl)ethyl group, a 1-(1-methoxycarbonylcyclopentyl)ethyl group;
a 1-(cyclohexyl)ethyl group, a 1-(1-methylcyclohexyl)ethyl group, a 1-(2-methylcyclohexyl)ethyl group, a 1-(3-methylcyclohexyl)ethyl group, a 1-(4-methylcyclohexyl)ethyl group, a 1-(2,3-dimethylcyclohexyl)ethyl group, a 1-(1-hydroxycyclohexyl)ethyl group, a 1-(2-hydroxycyclohexyl)ethyl group, a 1-(3-hydroxycyclohexyl)ethyl group, a 1-(4-hydroxycyclohexyl)ethyl group, a 1-(1-fluorocyclohexyl)ethyl group, a 1-(1-chlorocyclohexyl)ethyl group, a 1-(1-cyanocyclohexyl)ethyl group, a 1-(1-ethoxycarbonylcyclohexyl)ethyl group, a 1-(1-methoxycarbonylcyclohexyl)ethyl group, a 1-(1-ethynylcyclohexyl)ethyl group, a 1-(2-fluorocyclohexyl)ethyl group, a 1-(2-chlorocyclohexyl)ethyl group, a 1-(2-cyanocyclohexyl)ethyl group, a 1-(2-ethoxycarbonylcyclohexyl)ethyl group, a 1-(2-methoxycarbonylcyclohexyl)ethyl group;
a 1-methyl-1-cyclopropylethyl group, a 1-methyl-1-cyclobutylethyl group, a 1-methyl-1-(1-hydroxycyclobutyl)ethyl group, a 1-methyl-1-(1-fluorocyclobutyl)ethyl group, a 1-methyl-l-cyclopentylethyl group, a 1-methyl-1-(1-hydrdoxycylcopentyl)ethyl group, a 1-methyl-1-(1-tluorocyclopentyl)ethyl group, a 1-methyl-1-cyclohexylethyl group, a 1-methyl-1-(1-hydroxycyclohexyl)ethyl group and a 1-methyl-1-(1-fluorocyclohexyl)ethyl group.

Examples of the compound of the present invention include the following embodiments:
an amide compound of the formula (1), wherein Z is an oxygen atom, X¹ is a fluorine atom, and X² is a chlorine atom;
an amide compound of the formula (1), wherein Z is an oxygen atom, X¹ is a fluorine atom, and X² is a bromine atom;
an amide compound of the formula (1), wherein Z is an oxygen atom, X¹ is a fluorine atom, and X² is an iodine atom;
an amide compound of the formula (1), wherein Z is an oxygen atom, X¹ is a fluorine atom, and X² is a C₁-C₄ alkyl group;
an amide compound of the formula (1), wherein Z is an oxygen atom, X¹ is a fluorine atom, and X² is a C₂-C₄ alkenyl group;
an amide compound of the formula (1), wherein Z is an oxygen atom, X¹ is a fluorine atom, and X² is a C₂-C₄ alkynyl group;
an amide compound of the formula (1), wherein Z is an oxygen atom, X¹ is a fluorine atom, and X² is a C₁-C₄ haloalkyl group;
an amide compound of the formula (1), wherein Z is an oxygen atom, X¹ is a fluorine atom, and X² is a C₂-C₄ alkoxy group;
an amide compound of the formula (1), wherein Z is an oxygen atom, X¹ is a fluorine atom, and X² is a C₁-C₄ alkylthio group;
an amide compound of the formula (1), wherein Z is an oxygen atom, X¹ is a fluorine atom, and X² is a hydroxy C₁-C₄ alkyl group;
an amide compound of the formula (1), wherein Z is an oxygen atom, X¹ is a fluorine atom, and X² is a nitro group;
an amide compound of the formula (1), wherein Z is an oxygen atom, X¹ is a fluorine atom, and X² is a cyano group;
an amide compound of the formula (1), wherein Z is an oxygen atom, X¹ is a fluorine atom, and X² is a formyl group;
an amide compound of the formula (1), wherein Z is an oxygen atom, X¹ is a fluorine atom, and X² is an NR¹R² group;
an amide compound of the formula (1), wherein Z is an oxygen atom, X¹ is a fluorine atom, and X² is a CO₂R³ group;
an amide compound of the formula (1), wherein Z is an oxygen atom, X¹ is a fluorine atom, and X² is a CONR⁴R⁵ group;
an amide compound of the formula (1), wherein Z is an oxygen atom, X¹ is a fluorine atom, and X² is a phenyl group optionally substituted with at least one group selected from the group consisting of a methyl group, a halogen atom, a cyano group and a nitro group;

an amide compound of the formula (1), wherein Z is an oxygen atom, X¹ is a methoxy group, and X² is a chlorine atom;
an amide compound of the formula (1), wherein Z is an oxygen atom, X¹ is a methoxy group, and X² is a bromine atom;
an amide compound of the formula (1), wherein Z is an oxygen atom, X¹ is a methoxy group, and X² is an iodine atom;
an amide compound of the formula (1), wherein Z is an oxygen atom, X¹ is a methoxy group, and X² is a C₁-C₄ alkyl group;
an amide compound of the formula (1), wherein Z is an oxygen atom, X¹ is a methoxy group, and X² is a C₂-C₄ alkenyl group;
an amide compound of the formula (1), wherein Z is an oxygen atom, X¹ is a methoxy group, and X² is a C₂-C₄ alkynyl group;
an amide compound of the formula (1), wherein Z is an oxygen atom, X¹ is a methoxy group, and X² is a C₁-C₄ haloalkyl group;
an amide compound of the formula (1), wherein Z is an oxygen atom, X¹ is a methoxy group, and X² is a C₂-C₄ alkoxy group;
an amide compound of the formula (1), wherein Z is an oxygen atom, X¹ is a methoxy group, and X² is a C₁-C₄ alkylthio group;
an amide compound of the formula (1), wherein Z is an oxygen atom, X¹ is a methoxy group, and X² is a hydroxy C₁-C₄ alkyl group;
an amide compound of the formula (1), wherein Z is an oxygen atom, X¹ is a methoxy group, and X² is a nitro group;
an amide compound of the formula (1), wherein Z is an oxygen atom, X¹ is a methoxy group, and X² is a cyano group;
an amide compound of the formula (1), wherein Z is an oxygen atom, X¹ is a methoxy group, and X² is a formyl group;
an amide compound of the formula (1), wherein Z is an oxygen atom, X¹ is a methoxy group, and X² is an NR¹R² group;
an amide compound of the formula (1), wherein Z is an oxygen atom, X¹ is a methoxy group, and X² is a CO₂R³ group;
an amide compound of the formula (1), wherein Z is an oxygen atom, X¹ is a methoxy group, and X² is a CONR⁴R⁵ group;
an amide compound of the formula (1), wherein Z is an oxygen atom, X¹ is a methoxy group, and X² is a phenyl group optionally substituted with at least one group selected from the group consisting of a methyl group, a halogen atom, a cyano group and a nitro group;

an amide compound of the formula (1), wherein Z is an oxygen atom;
an amide compound of the formula (1), wherein X¹ is a fluorine atom;
an amide compound of the formula (1), wherein X¹ is a methoxy group;
an amide compound of the formula (1), wherein Z is an oxygen atom, and X¹ is a fluorine atom;
an amide compound of the formula (1), wherein Z is an oxygen atom, and X¹ is a methoxy group;
an amide compound of the formula (1), wherein X² is a chlorine atom, a bromine atom or a C₁-C₄ alkyl group;
an amide compound of the formula (1), wherein X² is a chlorine atom or a C₁-C₄ alkyl group;
an amide compound of the formula (1), wherein X² is a chlorine atom;
an amide compound of the formula (1), wherein X² is a C₁-C₄ alkyl group;
an amide compound of the formula (1), wherein Z is an oxygen atom, and X² is a chlorine atom or a C₁-C₄ alkyl group;
an amide compound of the formula (1), wherein Z is an oxygen atom, and X² is a chlorine atom;
an amide compound of the formula (1), wherein Z is an oxygen atom, and X² is a C₁-C₄ alkyl group;

an amide compound of the formula (1), wherein A is A¹-CR¹¹R¹²R¹³;
an amide compound of the formula (1), wherein A is A¹-CR¹¹R¹²R¹³, R¹¹ is a methyl group or an ethyl group, and R¹² is a methyl group, an ethyl group, a 1-methylethyl group, a 1,1-dimethylethyl group or a 1-methylpropyl group;
an amide compound of the formula (1), wherein A is A¹-CR¹¹R¹²R¹³, and R¹³ is a hydrogen atom or a methyl group;
an amide compound of the formula (1), wherein A is A¹-CR¹¹R¹²R¹³, A¹ is a single bond, R¹¹ is a methyl group, R¹² is a 1-methylethyl group or a 1,1-dimethylethyl group, and R¹³ is a hydrogen atom;
an amide compound of the formula (1), wherein A is A²-Cy;
an amide compound of the formula (1), wherein A is A²-Cy, and A² is a single bond;
an amide compound of the formula (1), wherein A is A²-Cy, and A² is a CH₂ group;
an amide compound of the formula (1), wherein A is A²-Cy, and A² is a CH (CH₃) group;

an amide compound of the formula (1), wherein A is A²-Cy, and Cy is a C₃-C₆ cycloalkyl group optionally substituted with at least one group selected from the group consisting of a C₁-C₄ alkyl group, a halogen atom, a hydroxyl group and a cyano group;
an amide compound of the formula (1), wherein A is A²-Cy, and Cy is a cyclopropyl group optionally substituted with at least one group selected from the group consisting of a C₁-C₄ alkyl group, a halogen atom, a hydroxyl group and a cyano group;
an amide compound of the formula (1), wherein A is A²-Cy, and Cy is a cyclobutyl group optionally substituted with at least one group selected from the group consisting of a C₁-C₄ alkyl group, a halogen atom, a hydroxyl group and a cyano group;
an amide compound of the formula (1), wherein A is A²-Cy, and Cy is a cyclopentyl group optionally substituted with at least one group selected from the group consisting of a C₁-C₄ alkyl group, a halogen atom, a hydroxyl group and a cyano group;
an amide compound of the formula (1), wherein A is A²-Cy, and Cy is a cyclohexyl group optionally substituted with at least one group selected from the group consisting of a C₁-C₄ alkyl group, a halogen atom, a hydroxyl group and a cyano group;

an amide compound of the formula (1), wherein A is A²-Cy, A² is a single bond, and Cy is a cyclohexyl group optionally substituted with at least one group selected from the group consisting of a methyl group, a halogen atom and a hydroxyl group;
an amide compound of the formula (1), wherein A is A²-Cy, A² is a CH₂ group, and Cy is a cyclohexyl group optionally substituted with at least one group selected from the group consisting of a methyl group, a halogen atom and a hydroxyl group;
an amide compound of the formula (1), wherein A is A²-Cy, A² is a CH(CH₃) group, and Cy is a cyclohexyl group optionally substituted with at least one group selected from the group consisting of a methyl group, a halogen atom and a hydroxyl group;
an amide compound of the formula (1), wherein A is A²-Cy, A² is a single bond, and Cy is a cyclopentyl group optionally substituted with at least one group selected from the group consisting of a methyl group, a halogen atom and a hydroxyl group;
an amide compound of the formula (1), wherein A is A²-Cy, A² is a CH₂ group, and Cy is a cyclopentyl group optionally substituted with at least one group selected from the group consisting of a methyl group, a halogen atom and a hydroxyl group;
an amide compound of the formula (1), wherein A is A²-Cy, A² is a CH(CH₃) group, and Cy is a cyclopentyl group optionally substituted with at least one group selected from the group consisting of a methyl group, a halogen atom and a hydroxyl group;
an amide compound of the formula (1), wherein A is A²-Cy, A² is a single bond, and Cy is a cyclobutyl group optionally substituted with at least one group selected from the group consisting of a methyl group, a halogen atom and a hydroxyl group;
an amide compound of the formula (1), wherein A is A²-Cy, A² is a CH₂ group, and Cy is a cyclobutyl group optionally substituted with at least one group selected from the group consisting of a methyl group, a halogen atom and a hydroxyl group;
an amide compound of the formula (1), wherein A is A²-Cy, A² is a CH(CH₃) group, and Cy is a cyclobutyl group optionally substituted with at least one group selected from the group consisting of a methyl group, a halogen atom and a hydroxyl group;

an amide compound of the formula (1), wherein A is A²-Cy, and Cy is a C₄-C₆ cycloalkyl group optionally substituted with a methyl group;
an amide compound of the formula (1), wherein A is A²-Cy, and Cy is a C₄-C₆ cycloalkyl group optionally substituted with a halogen atom;
an amide compound of the formula (1), wherein A is A²-Cy, and Cy is a C₄-C₆ cycloalkyl group optionally substituted with a fluorine atom;
an amide compound of the formula (1), wherein A is A²-Cy, and Cy is a C₄-C₆ cycloalkyl group optionally substituted with a chlorine atom;
an amide compound of the formula (1), wherein A is A²-Cy, and Cy is a C₄-C₆ cycloalkyl group optionally substituted with a hydroxyl group;
an amide compound of the formula (1), wherein A is A²-Cy, and Cy is a C₄-C₆ cycloalkyl group optionally substituted with a halogen atom;
an amide compound of the formula (1), wherein A is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group, a 2-hydroxycyclohexyl group, a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group, a 1-cyclopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group;
an amide compound of the formula (1), wherein A is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group or a 2-hydroxycyclohexyl group;
an amide compound of the formula (1), wherein A is a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group, a 1-cyclopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group;

an amide compound of the formula (1), wherein X² is a chlorine atom or a C₁-C₄ alkyl group, and A is A²-Cy;
an amide compound of the formula (1), wherein X² is a chlorine atom or a C₁-C₄ alkyl group, A is A²-Cy, and A² is a single bond or a CH₂ group;
an amide compound of the formula (1), wherein X² is a chlorine atom or a C₁-C₄ alkyl group, A is A²-Cy, A² is a single bond or a CH₂ group, and Cy is a C₃-C₆ cycloalkyl group optionally substituted with at least one group selected from the group consisting of a C₁-C₄ alkyl group and a hydroxyl group;
an amide compound of the formula (1), wherein X² is a chlorine atom or a C₁-C₄ alkyl group, A is A²-Cy, A² is a single bond or a CH₂ group, and Cy is a C₅-C₆ cycloalkyl group optionally substituted with at least one group selected from the group consisting of a C₁-C₄ alkyl group and a hydroxyl group;
an amide compound of the formula (1), wherein Z is an oxygen atom, X² is a chlorine atom or a C₁-C₄ alkyl group, A is A²-Cy, and A² is a single bond or a CH₂ group;
an amide compound of the formula (1), wherein Z is an oxygen atom, X² is a chlorine atom or a C₁-C₄ alkyl group, A is A²-Cy, A² is a single bond or a CH₂ group, and Cy is a C₃-C₆ cycloalkyl group optionally substituted with at least one group selected from the group consisting of a C₁-C₄ alkyl group and a hydroxyl group;
an amide compound of the formula (1), Z is an oxygen atom, X² is a chlorine atom or a C₁-C₄ alkyl group, A is A²-Cy, A² is a single bond or a CH₂ group, and Cy is a C₅-C₆ cycloalkyl group optionally substituted with at least one group selected from the group consisting of a C₁-C₄ alkyl group and a hydroxyl group;

an amide compound of the formula (1), wherein A is a 2-methylcyclohexyl group;
an amide compound of the formula (1), wherein A is a 2-chlorocyclohexyl group;
an amide compound of the formula (1), wherein A is a cyclohexylmethyl group;
an amide compound of the formula (1), wherein A is a (1-hydroxycyclohexyl)methyl group;
an amide compound of the formula (1), wherein A is a 1-(1-hydroxycyclohexyl)ethyl group;
an amide compound of the formula (1), wherein A is a cyclobutylmethyl group;
an amide compound of the formula (1), wherein A is a (1-hydroxycyclobutyl)methyl group;
an amide compound of the formula (1), wherein A is a 1-(1-hydroxycyclobutyl)ethyl group;
an amide compound of the formula (1), wherein Z is an oxygen atom, and A is a 2-methylcyclohexyl group;
an amide compound of the formula (1), wherein Z is an oxygen atom, and A is a 2-chlorocyclohexyl group;
an amide compound of the formula (1), wherein Z is an oxygen atom, and A is a cyclohexylmethyl group;
an amide compound of the formula (1), wherein Z is an oxygen atom, and A is a (1-hydroxycyclohexyl)methyl group;
an amide compound of the formula (1), wherein Z is an oxygen atom, and A is a 1-(1-hydroxycyclohexyl)ethyl group;
an amide compound of the formula (1), wherein Z is an oxygen atom, and A is a cyclobutylmethyl group;
an amide compound of the formula (1), wherein Z is an oxygen atom, and A is a (1-hydroxycyclobutyl)methyl group; and
an amide compound of the formula (1), wherein Z is an oxygen atom, and A is a 1-(1-hydroxycyclobutyl)ethyl group.

In the specification, a structural formula of a compound may conveniently represent a specific isomer. However, the present invention includes all active isomers such as geometrical isomers, optical isomers, steric isomers and tautomers which may be generated due to the structure of a compound, and a mixture of isomers. In the present invention, a compound is not limited to the description of a specific formula conveniently described and may be any one of isomers or a mixture of isomers. Therefore, in the present invention, a compound may have an asymmetric carbon atom in its molecule and thus may exist as an optically active form or a racemate. In the present invention, there is no limitation, and all cases are included.

Then, a process for producing the compound of the present invention is explained.
The compound of the present invention can be produced, for example, by the following Production process 1 to Production process 7.

### (Production process 1)

Of compounds of the present invention, a compound (5) of the present invention in which Z is an oxygen atom can be produced by reacting a compound (2) and a compound (3) in the presence of a dehydration condensing agent. 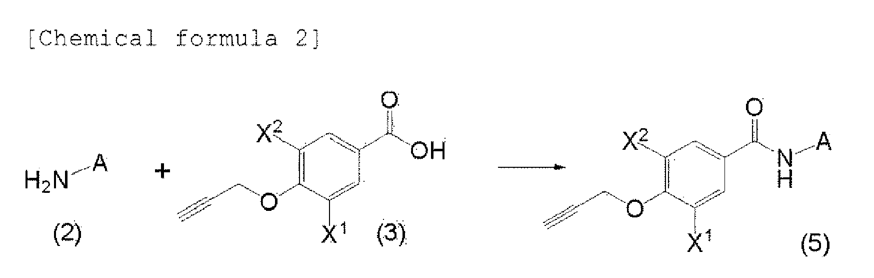 In the formulae, A, X¹ and X² are as defined above.
The reaction is usually performed in the presence of a solvent.
Examples of the solvent used in the reaction include ethers such as tetrahydrofuran (hereinafter, referred to as THF in some cases), ethylene glycol dimethyl ether and tert-butyl methyl ether (hereinafter, referred to as MTBE in some cases), aliphatic hydrocarbons such as hexane, heptane and octane, aromatic hydrocarbons such as toluene and xylene, halogenated hydrocarbons such as chlorobenzene, esters such as butyl acetate and ethyl acetate, nitriles such as acetonitrile, acid amides such as N,N-dimethylformamide (hereinafter, referred to as DMF in some cases), sulfoxides such as dimethyl sulfoxide (hereinafter, referred to as DMSO in some cases), and a mixture thereof.
Examples of the dehydration condensing agent used in the reaction include carbodiimides such as 1-ethyl-3-(3-dimethylaminopropyl)carbodiimide hydrochloride (hereinafter, referred to as WSC) and 1,3-dicyclohexylcarbodiimide.
The used amount of the compound (3) is usually 1 to 3 mol per 1 mol of the compound (2). The used amount of the dehydration condensing agent is usually 1 to 5 mol per 1 mol of the compound (2).
The reaction temperature is usually in a range of from 0 to 140°C, and the reaction time is usually in a range of from 1 to 24 hours.
After completion of the reaction, the compound (5) of the present invention can be isolated by post-treatment such as filtration of the reaction mixture, extraction of the filtrate with an organic solvent, and drying and concentration of an organic layer.
The isolated compound (5) of the present invention can be further purified by chromatography, recrystallization or the like.

### (Production process 2)

Of compounds of the present invention, a compound (5) of the present invention in which Z is an oxygen atom can be produced by reacting a compound (2) and a compound (4) in the presence of a base. 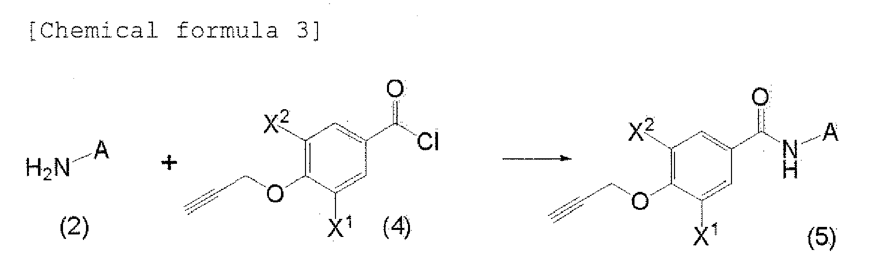 In the formulae, A, X¹ and X² are as defined above.
The reaction is usually performed in the presence of a solvent.
Examples of the solvent used in the reaction include ethers such as THF, ethylene glycol dimethyl ether and MTBE, aliphatic hydrocarbons such as hexane, heptane and octane, aromatic hydrocarbons such as toluene and xylene, halogenated hydrocarbons such as chlorobenzene, esters such as butyl acetate and ethyl acetate, nitriles such as acetonitriles, acid amides such as DMF, sulfoxides such as DMSO, and a mixture thereof.
Examples of the base used in the reaction include alkali metal carbonates such as sodium carbonate and potassium carbonate, tertiary amines such as triethyl amine and diisopropylethylamine, and nitrogen-containing aromatic compounds such as pyridine and 4-dimethylaminopyridine.
The used amount of the compound (4) is usually 1 to 3 mol per 1 mol of the compound (2). The used amount of the base is usually 1 to 10 mol per 1 mol of the compound (2).
The reaction temperature is usually in a range of from -20 to 100°C, and the reaction time is usually in a range of from 0.1 to 24 hours.
After completion of the reaction, the compound (5) of the present invention can be isolated by post-treatment such as filtration of the reaction mixture, extraction of the filtrate with an organic solvent, and drying and concentration of an organic layer.
The isolated compound (5) of the present invention can be further purified by chromatography, recrystallization or the like.

### (Production process 3)

Of compounds of the present invention, a compound (6) of the present invention in which Z is a sulfur atom can be produced by reacting the compound (5) of the present invention in which Z is an oxygen atom and 2,4-bis(4-methoxyphenyl)-1,3-dithia-2,4-diphosphethane-2,4-disulfide (hereinafter, referred to as Lawesson's reagent). 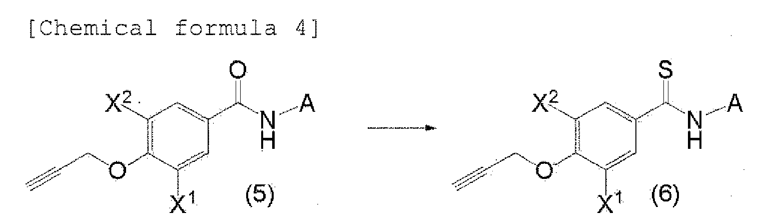 In the formulae, A, X¹ and X² are as defined above.
The reaction is usually performed in the presence of a solvent.
Examples of the solvent used in the reaction include ethers such as THF, ethylene glycol dimethyl ether and MTBE, aliphatic hydrocarbons such as hexane, heptane and octane, aromatic hydrocarbons such as toluene and xylene, halogenated hydrocarbons such as chlorobenzene, organic nitriles such as acetonitrile and butyronitrile, sulfoxides such as dimethyl sulfoxide, and a mixture thereof.
The used amount of the Lawesson's reagent is usually 1 to 2 mol per 1 mol of the compound (5) of the present invention.
The reaction temperature is usually in a range of from 25 to 150°C, and the reaction time is a usually in a range of from 0.1 to 24 hours.
After completion of the reaction, the compound (6) of the present invention can be isolated by post-treatment such as extraction of the reaction mixture with an organic solvent, and drying and concentration of an organic layer. The isolated compound (6) of the present invention can be further purified by chromatography, recrystallization or the like.

### (Production process 4)

Of compounds of the present invention, a compound (9) of the present invention in which Z is an oxygen atom and X¹ is a fluorine atom can be produced by reacting a compound (7) and a compound (2) in the presence of a base to obtain a compound (8) (step (IV-1)) and then reacting the compound (8) and propargyl alcohol in the presence of a base (step (IV-2)). 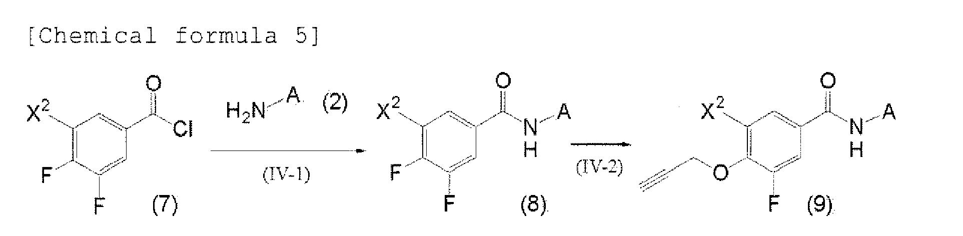 In the formulae, A and X² are as defined above.

### Step (IV-1)

The reaction is usually performed in the presence of a solvent.
Examples of the solvent used in the reaction include ethers such as THF, ethylene glycol dimethyl ether and MTBE, aliphatic hydrocarbons such as hexane, heptane and octane, aromatic hydrocarbons such as toluene and xylene, halogenated hydrocarbons such as chlorobenzene, esters such as butyl acetate and ethyl acetate, nitriles such as acetonitrile, acid amides such as DMF, sulfoxides such as dimethyl sulfoxide, and a mixture thereof.
Examples of the base used in the reaction include alkali metal carbonates such as sodium carbonate and potassium carbonate, tertiary amines such as triethylamine and diisopropylethylamine, and nitrogen-containing aromatic compounds such as pyridine and 4-dimethylaminopyridine.
The used amount of the compound (7) is usually 1 to 3 mol per 1 mol of the compound (2). The used amount of the base usually 1 to 10 mol per 1 mol of the compound (2).
The reaction temperature is usually in a range of from -20 to 100°C, and the reaction time is usually in a range of from 0.1 to 24 hours.
After completion of the reaction, the compound (8) can be isolated by post-treatment such as extraction of the reaction mixture with an organic solvent, and drying and concentration of an organic layer. The isolated compound (8) can be further purified by chromatography, recrystallization or the like.

### Step (IV-2)

The reaction is usually performed in the presence of a solvent.
Examples of the solvent used in the reaction include ethers such as THF, ethylene glycol dimethyl ether and MTBE, aliphatic hydrocarbons such as hexane, heptane and octane, ketones such as acetone, methyl ethyl ketone and methyl isobutyl ketone, aromatic hydrocarbons such as toluene and xylene, halogenated hydrocarbons such as chlorobenzene, esters such as butyl acetate and ethyl acetate, nitriles such as acetonitrile, acid amides such as DMF, sulfoxides such as dimethyl sulfoxide, and a mixture thereof.
Examples of the base used in the reaction include alkali metal carbonates such as sodium carbonate and potassium carbonate, alkali metal hydrogen carbonates such as sodium hydrogen carbonate, alkali metal hydrides such as sodium hydride, and alkali metal hydroxides such as sodium hydroxide.
The used amount of propargyl alcohol is usually 1 to 3 mol per 1 mol of the compound (8). The used amount of the base is usually 1 to 2 mol per 1 mol of the compound (8).
The reaction temperature is usually in a range of from -20 to 100°C, and the reaction time is usually in a range of from 0.1 to 24 hours.
After completion of the reaction, the compound (9) of the present invention can be isolated by post-treatment such as extraction of the reaction mixture with an organic solvent, and drying and concentration of an organic layer. The isolated compound (9) of the present invention can be further purified by chromatography, recrystallization or the like.

### (Production process 5)

Of compounds of the present invention, a compound (5) of the present invention in which Z is an oxygen atom can be produced by reacting a compound (10) and propargyl bromide in the presence of a base. 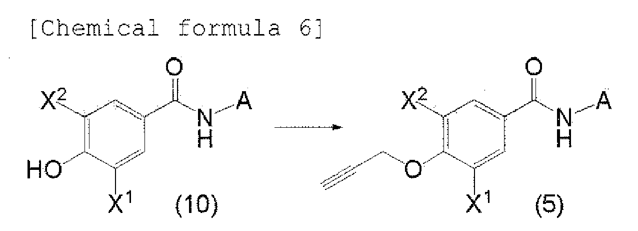 In the formulae, A, X¹ and X² are as defined above.
The reaction is usually performed in the presence of a solvent.
Examples of the solvent used in the reaction include ethers such as THF, ethylene glycol dimethyl ether and MTBE, aromatic hydrocarbons such as toluene and xylene, halogenated hydrocarbons such as chlorobenzene, nitriles such as acetonitrile, acid amides such as DMF, sulfoxides such as dimethyl sulfoxide, ketones such as acetone, methyl ethyl ketone and methyl isobutyl ketone, water, and a mixture thereof.
Examples of the base used in the reaction include alkali metal carbonates such as sodium carbonate, potassium carbonate and cesium carbonate, alkali metal hydroxides such as sodium hydroxide, and alkali metal hydrides such as sodium hydride.
The used amount of propargyl bromide is usually 1 to 3 mol per 1 mol of the compound (10). The used amount of the base is usually 1 to 3 mol per 1 mol of the compound (10).
The reaction temperature is usually in a range of from -20 to 100°C, and the reaction time is usually in a range of from 0.1 to 24 hours.
After completion of the reaction, the compound (5) of the present invention can be isolated by post-treatment such as extraction of the reaction mixture with an organic solvent, and drying and concentration of an organic layer. The isolated compound (5) of the present invention can be further purified by chromatography, recrystallization or the like.

### (Production process 6)

Of compounds of the present invention, a compound (12) of the present invention in which Z is an oxygen atom, A is A²-Cy, A² is a single bond, and Cy is a 2-fluorocyclohexyl group, a 2-chlorocyclohexyl group, a 2-bromocyclohexyl group, a 2-iodocyclohexyl group or a 2-cyanocyclohexyl group can be produced by a process shown in the following scheme. In addition, a compound (12) of the present invention in which R¹⁴ is a chlorine atom can be produced by reacting a compound (4) and 7-azabicyclo[4.1.0]heptane without isolation of an intermediate (11). 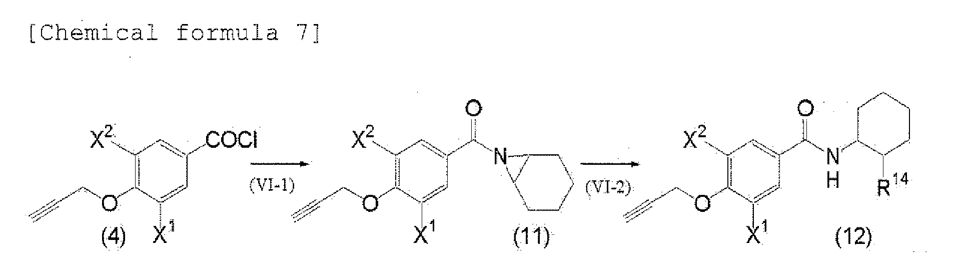 In the formulae, X¹ and X² are as defined above, and R¹⁴ represents a fluorine atom, a chlorine atom, a bromine atom, an iodine atom or a cyano group.

### Step (VI-1)

The compound (11) can be produced by reacting the compound (4) and 7-azabicyclo[4.1.0]heptane in the presence of a base according to the process described in Production process 2.

### Step (VI-2)

The compound (12) of the present invention can be produced by reacting the compound (11) and the following reagent.
Examples of the reagent used in the reaction, in the case where R¹⁴ in the compound (12) is a fluorine atom, include alkali metal fluorides such as potassium fluoride and lithium fluoride, alkaline earth metal fluorides such as calcium fluoride, quaternary ammonium fluorides such as tetrabutylammonium fluoride, and hydrogen fluoride.
Examples of the reagent used in the reaction, in the case where R¹⁴ in the compound (12) is a chlorine atom, include a alkali metal chlorides such as sodium chloride and lithium chloride, alkaline earth metal chlorides such as magnesium chloride, metal chlorides such as aluminum chloride and zinc(II) chloride, quaternary ammonium chlorides such as tetrabutylammonium chloride, organic silicon chlorides such as trimethylsilyl chloride, sulfur compounds such as thionyl chloride, phosphorus compounds such as phosphorus oxychloride, phosphorus trichloride and phosphorus pentachloride, and hydrogen chloride.
Examples of the reagent used in the reaction, in the case where R¹⁴ in the compound (12) is a bromine atom, include alkali metal bromides such as sodium bromide, alkaline earth metal bromides such as magnesium bromide, metal bromides such as zinc(II) bromide, quaternary ammonium bromides such as tetrabutylammonium bromide, organic silicon bromides such as trimethylsilyl bromide, phosphorus compounds such as phosphorus tribromide, and hydrogen bromide.
Examples of the reagent used in the reaction, in the case where R¹⁴ in the compound (12) is an iodine atom, include alkali metal iodides such as potassium iodide, alkaline earth metal iodides such as magnesium iodide, metal iodides such as zinc(II) iodide, quaternary ammonium iodides such as tetrabutylammonium iodide, organic silicon compounds such as trimethylsilyl iodide, and hydrogen iodide.
Examples of the reagent used in the reaction, in the case where R¹⁴ in the compound (12) is a cyano group, include cyanides such as potassium cyanide and sodium cyanide, and organic silicon compounds such as trimethylsilyl cyanide.
The reaction is usually performed in the presence of a solvent.
Examples of the solvent used in the reaction include ethers such as THF, ethylene glycol dimethyl ether and MTBE, aromatic hydrocarbons such as toluene and xylene, halogenated hydrocarbons such as chlorobenzene and chloroform, esters such as butyl acetate and ethyl acetate, nitriles such as acetonitrile, acid amides such as DMF, water, and a mixture thereof.
The used amount of the reagent is usually 1 to 10 mol per 1 mol of the compound (11).
The reaction temperature is usually in a range of from -20 to 150°C, and the reaction time is usually in a range of from 0.1 to 24 hours.
After completion of the reaction, the compound (12) of the present invention can be isolated by post-treatment such as extraction of the reaction mixture with an organic solvent, and drying and concentration of an organic layer. The isolated compound (12) of the present invention can be further purified by chromatography, recrystallization or the like.

### (Production process 7)

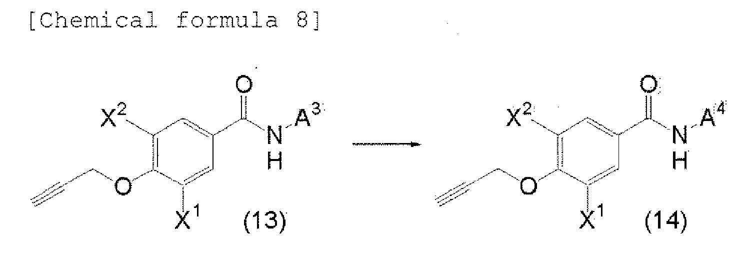

In the formulae, X¹ and X² are as defined above, A³ is A²-Cy and Cy represents a C₃-C₆ cycloalkyl group substituted with at least one hydroxyl group, and A⁴ is A²-Cy and Cy represents a C₃-C₆ cycloalkyl group substituted with at least one halogen atom.
The compound (14) of the present invention may be produced by reacting the compound (13) and the following halogenating reagent.
Examples of the halogenating reagent used in the reaction, in the case where the halogen atom of the C₃-C₆ cycloalkyl group substituted with at least one halogen atom in the compound (14) of the present invention is a fluorine atom, include fluorinating agents such as 2,2-difluoro-1,3-dimethylimidazolidine, hydrogen fluoride pyridine complex, diethyl(1,2,3,3,3-pentafluoro-1-propenyl)amine, di(2-methoxyethyl)sulfur trifluoride, diethylaminosulfur trifluoride, tetrafluorosulfur and N-(2-chloro-1,1,2-trifluoroethyl)diethylamine.
Examples of the halogenating reagent used in the reaction, in the case where the halogen atom of the C₃-C₆ cycloalkyl group substituted with at least one halogen atom in the compound (14) of the present invention is a chlorine atom, include chlorinating agents such as thionyl chloride, phosphorus oxychloride, phosphorus trichloride, phosphorus pentachloride and phosgene.
Examples of the halogenating reagent used in the reaction, in the case where the halogen atom of the C₃-C₆ cycloalkyl group substituted with at least one halogen atom in the compound (14) of the present invention is a bromine atom, include brominating agents such as phosphorus tribromide.
Examples of a solvent used in the reaction include ethers such as THF, ethylene glycol dimethyl ether and MTBE, aromatic hydrocarbons such as toluene and xylene, halogenated hydrocarbons such as chlorobenzene and chloroform, nitriles such as acetonitrile, and a mixture thereof.
The used amount of the reagent is usually 1 to 10 mol per 1 mol of the compound (13) of the present invention.
The reaction temperature is usually in a range of from -78 to 150°C, and the reaction time is usually in a range of from 0.1 to 24 hours.
After completion of the reaction, the compound (14) of the present invention can be isolated by post-treatment such as extraction of the reaction mixture with an organic solvent, and drying and concentration of an organic layer. The isolated compound (14) of the present invention can be further purified by chromatography, recrystallization or the like.

A part of intermediates used for production of the compound of the present invention are commercially available or disclosed in known literatures. Such intermediates can be produced, for example, by the following processes.

### (Intermediate production process 1)

The compound (3) and the compound (4) can be produced by the process shown in the following scheme. 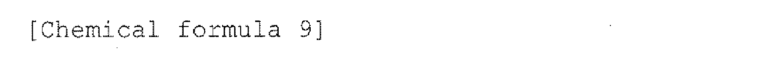 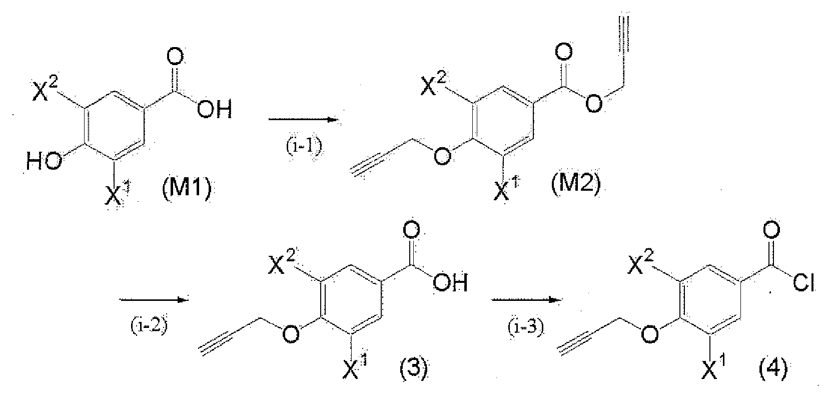 In the formulae, X¹ and X² are as defined above.

### Step (i-1)

The compound (M2) can be produced by reacting the compound (M1) and propargyl bromide in the presence of a base.
The reaction is usually performed in the presence of a solvent.
Examples of the solvent used in the reaction include acid amides such as DMF and sulfoxides such as DMSO.
Examples of the base used in the reaction include alkali metal carbonates such as sodium carbonate, potassium carbonate and cesium carbonate, and alkali metal hydroxides such as sodium hydroxide.
The used amount of propargyl bromide is usually 2 to 5 mol per 1 mol of the compound (M1). The used amount of the base is usually 2 to 5 mol per 1 mol of the compound (M1).
The reaction temperature is usually in a range of from 0 to 140°C, and the reaction time is usually in a range of from 0.5 to 24 hours.
After completion of the reaction, the compound (M2) can be isolated by post-treatment such as extraction of the reaction mixture with an organic solvent, and drying and concentration of an organic layer. The isolated compound (M2) can be further purified by chromatography, recrystallization or the like.

### Step (i-2)

The compound (3) can be produced by hydrolyzing the compound (M2) in the presence of a base.
The reaction is usually performed in the presence of a solvent.
Examples of the base used in the reaction include alkali metal hydroxides such as sodium hydroxide and potassium hydroxide.
Examples of the solvent used in the reaction include ethers such as tetrahydrofuran, ethylene glycol dimethyl ether and tert-butyl methyl ether, alcohols such as methanol and ethanol, water, and a mixture thereof.
The used amount of the base is usually 1 to 10 mol per 1 mol of the compound (M2).
The reaction temperature is usually in a range of from 0 to 120°C, and the reaction time is usually in a range of from 0.5 to 24 hours.
After completion of the reaction, the reaction solution is made acidic. In the case where a solid is precipitated, the compound (3) can be isolated by filtration. In the case where a solid is not precipitated, the compound (3) can be isolated by post-treatment such as extraction of the reaction mixture with an organic solvent, and drying and concentration of an organic layer. The isolated compound (3) can be further purified by chromatography, recrystallization or the like.

### Step (i-3)

The compound (4) can be produced by reacting the compound (3) and thionyl chloride.
The reaction is usually performed in the presence of a solvent.
Examples of the solvent used in the reaction include aliphatic hydrocarbons such as hexane, heptane and octane, aromatic hydrocarbons such as toluene and xylene, nitriles such as acetonitrile, halogenated hydrocarbons such as chlorobenzene, acid amides such as DMF, and a mixture thereof.
The used amount of thionyl chloride is usually 1 to 2 mol per 1 mol of the compound (3).
The reaction temperature is usually in a range of from 20 to 120°C, and the reaction time is usually in a range of from 0.1 to 24 hours.
After completion of the reaction, the compound (4) can be isolated by post-treatment such as concentration of the reaction mixture. The isolated compound (4) can be further purified by chromatography, recrystallization or the like.

### (Intermediate production process 2)

The compound (10) can be produced by the process shown in the following scheme. 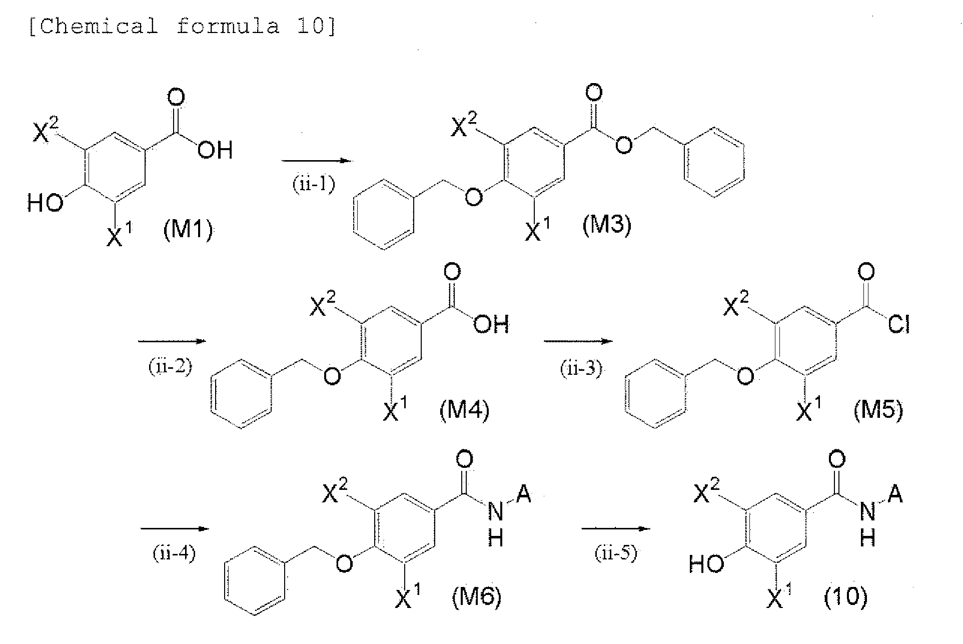 In the formulae, A, X¹ and X² are as defined above.

### Step (ii-1)

The compound (M3) can be produced by reacting the compound (M1) and benzyl bromide in the presence of a base.
The reaction is usually performed in the presence of a solvent.
Examples of the solvent used in the reaction include acid amides such as DMF, and sulfoxides such as DMSO.
Examples of the base used in the reaction include alkali metal carbonates such as sodium carbonate, potassium carbonate and cesium carbonate, and alkali metal hydroxides such as sodium hydroxide.
The used amount of benzyl bromide is usually 2 to 5 mol per 1 mol of the compound (M1). The used amount of the base is usually 2 to 5 mol per 1 mol of the compound (M1)
The reaction temperature is usually in a range of from 0 to 140°C, and the reaction time is usually in a range of from 0.5 to 24 hours.
After completion of the reaction, the compound (M3) can be isolated by post-treatment such as extraction of the reaction mixture with an organic solvent, and drying and concentration of an organic layer. The isolated compound (M3) can be further purified by chromatography, recrystallization or the like.

### Step (ii-2)

The compound (M4) can be produced by hydrolyzing the compound (M3) in the presence of a base.
The reaction is usually performed in the presence of a solvent.
Examples of the base used in the reaction include alkali metal hydroxides such as sodium hydroxide and potassium hydroxide.
Examples of the solvent used in the reaction include ethers such as tetrahydrofuran, ethylene glycol dimethyl ether and MTBE, alcohols such as methanol and ethanol, water, and a mixture thereof.
The used amount of the base is usually 1 to 10 mol per 1 mol of the compound (M3).
The reaction temperature is usually in a range of from 0 to 120°C, and the reaction time is usually in a range of from 0.5 to 24 hours.
After completion of the reaction, the reaction solution is made acidic. In the case where a solid is precipitated, the compound (M4) can be isolated by filtration. In the case where a solid is not precipitated, the compound (M4) can be isolated by post-treatment such as extraction of the reaction mixture with an organic solvent, and drying and concentration of an organic layer. The isolated compound (M4) can be further purified by chromatography, recrystallization of the like.

### Step (ii-3)

The compound (M5) can be produced by reacting the compound (M4) and thionyl chloride.
The reaction is usually performed in the presence of a solvent.
Examples of the solvent used in the reaction include aliphatic hydrocarbons such as hexane, heptane and octane, aromatic hydrocarbons such as toluene and xylene, nitriles such as acetonitrile, halogenated hydrocarbons such as chlorobenzene, acid amides such as DMF, and a mixture thereof.
The used amount of thionyl chloride is usually 1 to 2 mol per 1 mol of the compound (M4).
The reaction temperature is usually in a range of from 20 to 120°C, and the reaction time is usually in a range of from 0.1 to 24 hours.
After completion of the reaction, the compound (M5) can be isolated by post-treatment such as concentration of the reaction mixture. The isolated compound (M5) can be further purified by chromatography, recrystallization or the like.

### Step (ii-4)

The compound (M6) can be produced by reacting the compound (M5) and the compound (2) in the presence of a base according to the process described in Production process 2.

### Step (ii-5)

The compound (10) can be produced by reacting the compound (M6) with hydrogen in the presence of palladium carbon.
The reaction is usually performed in the presence of a solvent.
Examples of the solvent used in the reaction include aliphatic hydrocarbons such as hexane, heptane and octane, aromatic hydrocarbons such as toluene and xylene, alcohols such as methanol and ethanol, esters such as ethyl acetate, ethers such as THF and MTBE, water, and a mixture thereof.
The used amount of palladium carbon is 0.01 to 0.1 mol per 1 mol of the compound (M6). The used amount of hydrogen is usually 1 to 2 mol per 1 mol of the compound (M6).
The reaction temperature is usually in a range of from 0 to 50°C, and the reaction time is usually in a range of from 0.1 to 24 hours.
The pressure of hydrogen used in the reaction is in a range of from normal pressure to 10 atoms.
After completion of the reaction, the compound (10) can be isolated by post-treatment such as filtration and concentration of the reaction mixture. The isolated invented compound (10) can be further purified by chromatography, recrystallization or the like.

Examples of a compound represented by the formula (3): 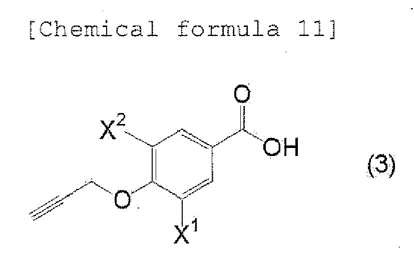 wherein X¹, and X² are as defined above, which is an intermediate for producing the compound of the present invention include the following compounds:
a compound of the formula (3), wherein X¹ is a fluorine atom; and
a compound of the formula (3), wherein X¹ is a methoxy group.

Examples of a compound represented by the formula (10) : 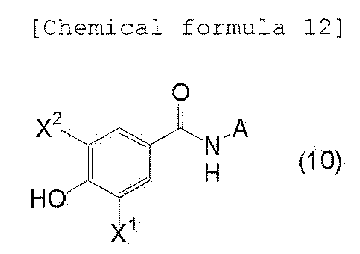 wherein X¹, X² and A are as defined above, which is an intermediate for producing the compound of the present invention include the following compounds:
a compound of the formula (10), wherein X¹ is a fluorine atom; and
a compound of the formula (10), wherein X¹ is a methoxy group.

Examples of a compound represented by the formula (11) : 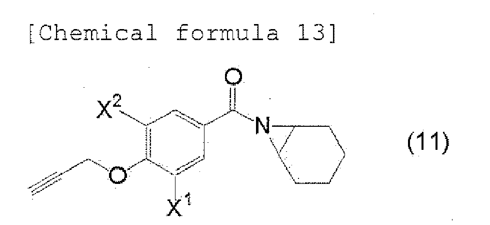 wherein X¹ and X² are as defined above, which is an intermediate for producing the compound of the present invention include the following compounds:
a compound of the formula (11), wherein X¹ is a fluorine atom; and
a compound of the formula (11), wherein X¹ is a methoxy group.

Plant diseases against which the compound of the present invention exerts an excellent effect include those caused by fungi, bacteria and viruses. Specific examples of the fungi include genus *Erysiphe* such as wheat powdery mildew (*Erysiphe graminis*), genus *Uncinula* such as grape powdery mildew (*Uncinula necator*), genus *Podosphaera* such as apple powdery mildew (*Podosphaera leucotricha*), genus *Sphaerotheca* such as cucumber powdery mildew (*Sphaerotheca cucurbitae*), genus *oidiopsis* such as tomato powdery mildew (*Oidiopsis sicula*), genus *Magnaporthe* such as rice blast (*Magnaporthe oryzae*), genus *Cachliobolus* such as rice brown spot (*Cochliobolus miyabeanus*), genus *Mycosphaerella* such as wheat leaf blotch (*Mycosphaerella graminicola*), genus *Pyrenophora* such as barley net blotch (*Pyrenophora* teres), genus *Stagonaspora* such as wheat Glume blotch (*Stagonaspara nodorum*), genus *Rhynchosporium* such as barley scald (*Rhynchosporium secalis*), genus *Pseudocercosporella* such as wheat eyespot (*Pseudocercosporella herpotrichoides*), genus *Gaeumannomyces* such as wheat take-all (*Gaeumannomyces graminis*), genus *Fusarium* such as wheat Fusarium, head blight (*Fusarium* spp.), genus *Microdochium* such as wheat snow mold (*Microdochium* nivale), genus Venturia such as apple scab (Venturia *inaequalis*), genus *Elsinoe* such as grape anthracnose (*Elsinoe ampelina*), genus *Botrytis* such as cucumber gray mold (*Botrytis cinerea*), genus *Monilinia* such as peach brown rot (*Monilinia fructicola*), genus Phoma such as rape stem canker (Phoma *lingam*), genus *Cladosporium* such as tomato leaf mold (*Cladosporium fulvum*), genus *Cercospora* such as sugarbeet brown spot (*Cercospora beticola*), genus *Cercosporidium* such as peanut late leaf spot (*Cercosporidium personatum*), genus *Colletotrichum* such as strawberry anthracnose (*Colletotrichum fragariae*), genus *Sclerotinia* such as cucumber stem rot (*Sclerotinia* sclerotiorum), genus Alternaria such as apple necrotic leaf spot (*Alternaria mali*), genus *Verticillium* such as eggplant verticillium wilt (*Verticillium* dahliae), genus Rhizoctonia such as rice sheath blight (*Rhizoctonia solani*), genus *Puccinia* such as wheat leaf rust (*Puccinia recondita*), genus *Phakopsora* such as soybean rust (*Phakopsora pachyrhizi*), genus *Tilletia* such as wheat bunt (*Tilletia caries),* genus *Ustilago* such as barley loose smut (*Ustilago nuda*), genus *Sclerotium* such as peanut southern blight (*Sclerotium rolfsii*), genus *Phytophthora* such as potato late blight (*Phytophthora infestans*), genus *Pseudoperonospora* such as cucumber downy mildew (*Pseudoperonospara cubensis*), genus *Peronospora* such as Chinese cabbage downy mildew (*Peronospora parasitica*), genus *Plasmopara* such as grape downy mildew (*Plasmopara viticola*), genus *Sclerophthora* such as rice downy mildew (*Sclerophthora macrospora*), genus Pythium such as cucumber seedling damping-off (*Pythium ultimum*), and genus *Plasmodiophora* such as rapeseed clubroot (*Plasmodiophora brassicae*). Examples of bacteria include genus *Burkholderia* such as bacterial rice seedling blight (*Burkholderia plantarii*), genus *Pseudomanas* such as bacterial cucumber leaf spot (*Pseudomonas syringae pv. Lachrymans*), genus *Ralstonia* such as eggplant wilting (*Ralstonia solanacearum*), genus *Xanthomonas* such as Asiatic citrus canker (*Xanthomonas citiri*), and genus Erwinia such as Chinese cabbage bacterial soft rot (*Erwinia carotovora*). Examples of viruses include Tobacco mosaic virus and Cucumber mosaic virus. However, the sterilizing spectra should not be limited thereto in any cases.

The plant disease controlling agent of the present invention can be the compound of the present invention itself, but usually it is used in the form of formulations such as emulsifiable concentrates, wettable powders, granular wettable powders, flowable formulations, dusts, and granules produced by mixing it with solid carriers, liquid carriers, surface active agents and other auxiliary agents for formulation. These formulations usually contain the compound of the present invention in an amount of 0.1 to 90 % by weight.

Examples of the solid carriers used in the formulations include fine powders or particles of minerals such as kaolin clay, attapulgite clay, bentonite, montmorillonite, acid clay, pyrophyllite, talc, diatomaceous earth, and calcite; natural organic substances such as corncob powder, and walnut shell flour; synthetic organic substances such as urea; salts such as calcium carbonate, and ammonium sulfate; and synthetic inorganic substances such as synthetic hydrated silicon oxide. Examples of the liquid carriers include aromatic hydrocarbons such as xylene, alkylbenzene and methylnaphthalene; alcohols such as 2-propanol, ethylene glycol, propylene glycol and cellosolve; ketones such as acetone, cyclohexanone and isophorone; vegetable oils such as soybean oil and cottonseed oil; petroleum aliphatic hydrocarbons; esters; and dimethyl sulfoxide, acetonitrile and water.

Examples of the surfactant include anionic surfactants such as alkyl sulfate, alkyl aryl sulfonate, dialkyl sulfosuccinate, polyoxyethylene alkyl aryl ether phosphate, ligninsulfonate and a naphthalenesulfonate formaldehyde polycondensate; and nonionic surfactants such as polyoxyethylene alkyl aryl ether, a polyoxyethylene alkyl polyoxypropylene block copolymer and sorbitan fatty acid ester.

Examples of the auxiliary agent for formulation include water-soluble polymers such as polyvinyl alcohol and polyvinyl pyrrolidone; polysaccharides such as gum Arabic, alginic acid and a salt thereof, CMC (carboxymethyl cellulose) and xanthan gum; inorganic substances such as aluminum magnesium silicate, and alumina sol; preservatives; colorants; PAP (acidic isopropyl phosphate); and stabilizers such as BHT.

The plant disease controlling agent of the present invention is used for treating plants to protect the plants from plant diseases, and is also used for treating soil to protect plants growing in the soil from plant diseases.

When the plant disease controlling agent of the present invention is used by subjecting plants to a foliage treatment used by treating soil, its application amount varies depending upon the kind of crops as plants to be protected, the kind of diseases to be controlled, severity of diseases, form of the formulation, time of application, weather conditions and the like. The total amount of the compound of the present invention is usually within a range of from 1 to 5,000 g, and preferably from 5 to 1,000 g per 10,000 m².

Emulsifiable concentrates, wettable powders and flowable formulations are usually used for treatment by spraying after dilution with water. In this case, the concentration of the compound of the present invention is usually within a range of from 0.0001 to 3% by weight, and preferably from 0.0005 to 1% by weight. Dusts and granules are usually used for a treatment without being diluted.

The plant disease controlling agent of the present invention can be used by a treating method such as seed disinfection. Examples of the method include a method of immersing seeds of plants in the plant disease controlling agent of the present invention in which the concentration of the compound of the present invention is adjusted within a range of 1 to 1,000 ppm, a method of spraying or smearing the plant disease controlling agent of the present invention in which the concentration of the compound of the present invention is adjusted within a range of from 1 to 1,000 ppm, on seeds of plants, and a method of dust coating of seeds of plants using the plant diseases controlling agent of the present invention.

The method for controlling a plant disease of the present invention is usually carried out by treating a plant in which onset of diseases is presumed, or soil where the plant is growing, with an effective amount of the plant disease controlling agent of the present invention, and/or treating a plant in which onset of diseases has been confirmed, or the soil where the plants are growing.

The plant disease controlling agent of the present invention can be used as a controlling agent for plant diseases in crop lands such as upland fields, paddy fields, lawn, and orchards, etc. The plant disease controlling agent can control plant diseases in crop lands where the following "crops" or the like are cultivated.

Field crops: corn, rice, wheat, barley, rye, oat, sorghum, cotton, soybean, peanut, buckwheat, beet, rape, sunflower, sugarcane, tobacco, etc.
Vegetables: solanaceae (e.g. eggplant, tomato, green pepper, pepper and potato), Cucurbitaceae (e.g. cucumber, pumpkin, zucchini, watermelon and melon), Cruciferae (e.g. Japanese radish, turnip, horseradish, kohlrabi, Chinese cabbage, cabbage, leaf mustard, broccoli and cauliflower), Compositae (e.g. edible burdock, garland chrysanthemum, globe artichoke and lettuce), Liliacede (e.g., Welsh onion, onion, garlic and asparagus), Umbelliferae (e.g. carrot, parsley, celery and pastinaca), Chenopodiaceae (e.g. spinach and chard), Lamiaceae (e.g. perilla, mint and basil), strawberry, sweet potato, Chinese yam, taro, etc.
Flowers and ornament plants.
Ornamental foliage plants.
Fruit trees: pomaceous fruits (e.g. apple, pear, Japanese pear, Chinese quince and quince), stone fruits (e.g. peach, plum, nectarine, Japanese apricot, cherry, apricot and prune), citrus fruits (e.g. satsuma mandarin, orange, lemon, lime and grapefruit), nut trees (e.g. chestnut, walnut, hazel, almond, pistachio, cashew nut and macadamia nut), berries (blueberry, cranberry, blackberry and raspberry), grape, Japanese persimmon, olive, loquat, banana, coffee, date palm, coconut palm, etc.
Trees other than fruit trees: tea, mulberry, flowering trees and shrubs, street trees (e.g. Japanese ash, birch, flowering dogwood, blue gum, ginkgo, lilac, maple, oak, poplar, Chinese redbud, Formosa sweet gum, plane tree, zelkova, Japanese arborvitae, fir, Japanese hemlock, needle juniper, pine, Japanese spruce and Japanese yew), etc.

The above-described "crops" include crops having resistance to herbicides such as HPPD inhibitors (e.g. isoxaflutole), ALS inhibitors (e.g. imazethapyr and thifensulfuron-methyl), EPSP synthetase inhibitors, glutamine synthetase inhibitors, bromoxynil, etc. which has been imparted by a classic breeding method or a genetic recombination technology.

Examples of the "crops" having the resistance imparted by the classic breeding method include Glearfield® canola resistnt to imidazolinone herbicides (e.g. imazethapyr) and STS soybean resistant to sulfonylurea ALS inhibition type herbicides (e.g. thifensulfuron-methyl). As crops having the resistance imparted by the genetic recombination technology, corn cultivars resistant to glyphosate and glufosinate are exemplified and are already on the market under the trade names of RoundupReady® and LibertyLink®.

The above-described "crops" include crops which have been enabled by the genetic recombination technology to synthesize a selective toxin known in the case of, for example Bacillus.
Examples of toxin produced in such genetically modified plants include insecticidal proteins derived from Bacillus cereus and Bacillus popilliae; insecticidal proteins such as δ-endotoxins (e.g. Cry1Ab, Cry1Ac, Cry1F, Cry1Fa2, Cry2Ab, Cry3A, Cry3Bb1 and Cry9C), VIP 1, VIP 2, VIP 3, VIP 3A, etc., which are derived from Bacillus thuringiensis; toxins derived from nematodes; toxins produced by animals, such as scorpion toxin, spider toxin, bee toxin, insect-specific neurotoxins, etc.; filamentous fungi toxins; plant lectins; agglutinin; protease inhibitors such as trypsin inhibitors, serine protease inhibitors, patatin, cystatin, papain inhibitors, etc.; ribosome-inactivating proteins (RIPs) such as ricin, corn-RIP, abrin, rufin, sapolin, briodin, etc.; steroid metabolic enzymes such as 3-hydroxysteroid oxidase, ecdysteroid-UDP-glucosyltransferase, cholesterol oxidase, etc.; ecdysone inhibitors; HMG-COA reductase; ion channel inhibitors such as sodium channel inhibitors, calcium channel inhibitors, etc.; juvenile hormone esterase; diuretic hormone receptors; stilbene synthetase; bibenzyl synthetase; chitinase; and glucanase.

The toxin produced in such genetically engineered crops also include hybrid toxins, partly deficient toxins and modified toxins of insecticidal proteins such as 5-endotoxin proteins (e.g. Cry1Ab, Cry1Ac, Cry1F, Cry1Fa2, Cry2Ab, Cry3A, Cry3Bb1 and Cry9C), VIP 1, VIP 2, VIP 3, VIP 3A, etc. The hybrid toxins are produced by a novel combination of the different domains of suchproteins by adoping a recombination technology. As the partly deficient toxin, Cry1Ab deficient in a part of the amino acid sequence is known. In the modified toxins, one or more amino acids of a natural toxin have been replaced.
Examples of such toxins and genetically modified plants capable of synthesizing such toxins are described in EP-A-0 374 753, WO 93/07278, WO 95/34656, EP-A-0 427 529, EP-A-451 878, WO 03/052073, etc.
The toxins contained in such genetically engineered plants impart resistance to insect pests of Coleoptera, insect pests of Diptera and insect pests of Lepidoptera to the plants.

Genetically engineered plants containing one or more insecticidal insect-resistance genes and capable of producing one or more toxins have already been known, and some of them are on the market.. Examples of such genetically modified plants include YieldGard® (a corn cultivar capable of producing Cry1Ab toxin), YieldGard Rootwarm® (a corn cultivar capable of producing Cry3Bb1 toxin), YieldGard Plus® (a corn cultivar capable of producing Cry1Ab and Cry3Bb1 toxins), Herculex I® (a corn cultivar capable of producing phosphinotrysin N-acetyltransferase (PAT) for imparting resistance to Cry1Fa2 toxin and Glyfosinate), NuCOTN33B (a cotton cultivar capable of producing Cry1Ac toxin), Bollgard I® (a cotton cultivar capable of producing Cry1Ac toxin), Bollgard II® (a cotton cultivar capable of producing Cry1Ac and Cry2Ab toxins), VIPCOT® (a cotton cultivar capable of producing VIP toxin), NewLeaf® (a potato cultivar capable of producing Cry3A toxin), NatureGard®, Agrisure®, GT Advantage (GA21 glyphosate resistant properties), Agrisure® CB Advantage (Bt11 corn borer (CB) properties), and Protecta®.

The above-mentioned "crops" also include crops having an ability to produce an anti-pathogenic substance having a selective action which has been imparted by a gene recombination technology.
As examples of the anti-pathogenic substance, PR proteins and the like are known (PRPs, EP-A-0 392 225). Such anti-pathogenic substances and genetically engineered plants capable of producing them are described in EP-A-0 392 225, WO 95/33818, EP-A-0 353 191, and the like.
Examples of such anti-pathogenic substances produced by the genetically modified plants include ion channel inhibitors such as sodium channel inhibitors, calcium channel inhibitors (for example, KP1, KP4 and KP6 toxins produced by viruses are known), etc.; stilbene synthase; bibenzyl synthase; chitinase; glucanase; PR proteins; and anti-pathogenic substances produced by microorganisms such as peptide antibiotics, antibiotics having a heterocyclic ring, protein factors concerned in resistance to plant diseases (which are called plant-disease-resistant genes and are described in WO 03/000906), etc.

It is also possible to use the plant disease controlling agent of the present invention after mixing with other fungicides, insecticides, acaricides, nematocides, herbicides, plant growth regulators, fertilizers or soil conditioners, or to use the agent without mixing them. Examples of the active ingredients of the plant disease controlling agent include chlorothalonil, fluazinam, dichlofluanid, fosetyl-Al, cyclic imide derivatives (e.g., captan, captafol, folpet, etc.), dithiocarbamate derivatives (e.g., maneb, mancozeb, thiuram, ziram, zineb, propineb, etc.), inorganic or organic copper derivatives (e.g., basic copper sulfate, basic copper chloride, copper hydroxide, oxine-copper, etc.), acylalanine derivatives (e.g., metalaxyl, furalaxyl, ofurace, cyprofuram, benalaxyl, oxadixyl, etc.), strobilurine like compound (e.g., kresoxim-methyl, azoxystrobin, trifloxystrobin, picoxystrobin, pyraclostrobin, fluoxastrobin, metominostrobin, orysastrobin, enestrobin, dimoxystrobin, etc.), anilinopyrimidine derivatives (e.g., cyprodinil, pyrimethanil, mepanipyrim, etc.), phenyl pyrrole derivatives (e.g., fenpiclonil, fludioxonil, etc.), imide derivatives (e.g., procymidone, iprodione, vinclozolin, etc.), benzimidazole derivatives (e.g., carbendazim, benomyl, thiabendazole, thiophanate methyl, etc.), amine derivatives (e.g., fenpropimorph, tridemorph, fenpropidin, spiroxamine, etc.), azole derivatives (e.g., propiconazole, triadimenol, prochloraz, penconazole, tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, flutriafol, ipconazole, pefurazoate, prothioconazole, etc.), triforine, pyrifenox, fenarimol, propamocarb, cymoxanil, dimethomorph, flumorph, famoxadone, fenamidone, pyribencarb, iprovalicarb, benthiavalicarb, mandipropamid, cyazofamid, amisulbrom, zoxamide, ethaboxam, boscalid, penthiopyrad, fluopyram, bixafen, carboxin, oxycarboxin, thifluzamide, flutolanil, mepronil, furametpyr, pencycuron, hymexazol, etridiazole, ferimzone, silthiofam, blasticidin S, kasugamycin, streptomycin, pyrazophos, iprobenfos, edifenphos, isoprothiolane, fthalide, pyroquilon, tricyclazole, carpropamid, diclocymet, fenoxanil, probenazole, tiadinil, isotianil, iminoctadine, guazatine, tolnifanide, tolclophos-methyl, fenhexamid, polyoxin B, quinoxyfen, proquinazid, metrafenone, cyflufenamid, diethofencarb, fluopicolide and acibenzolar-S-methyl.

### Examples

Hereinafter, the present invention will be described in more detail by way of Production Examples, Formulation Examples and Test Examples which the present invention is not limited to.
First, Production Examples of the compound of the present invention will be described.

### Production Example 1

To a mixture of 1 ml of ethyl acetate and 0.24 g of 3-chloro-5-fluoro-4-(2-propynyloxy)benzoyl chloride was added dropwise a mixture of 0.13 g of cyclohexylmethylamine, 0.2 ml of triethylamine and 1 ml of ethyl acetate under ice-cooling, and the mixture was stirred at room temperature for 4 hours. The reaction mixture was concentrated under reduced pressure. The residue was subjected to silica gel column chromatography, and 0.25 g of N-(cyclohexylmethyl)-3-chloro-5-fluoro-4-(2-propynyloxy)benzamide (hereinafter, referred to as a compound 1 of the present invention) was obtained.
The compound 1 of the present invention 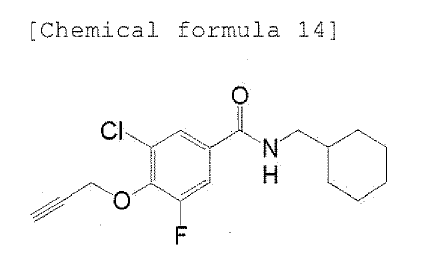 ¹H-NMR (CDCl₃) δ: 0.97-1.04 (2H, m), 1.15-1.30 (3H, m), 1.57-1.78 (6H, m), 2.51 (1H, t, J = 2.4 Hz), 3.28 (2H, dd, J = 6.6, 6.3 Hz), 4.88 (2H, d, J = 2.4 Hz), 6.03-6.06 (1H, m), 7.47 (1H, dd, J = 11.0, 1.9 Hz), 7.56 (1H, dd, J = 2.1, 1.9 Hz).

### Production Example 2

N-(2-methylcyclohexyl)-3-chloro-5-fluoro-4-(2-propynyloxy)benzamide (hereinafter, referred to as a compound 2 of the present invention) was obtained according to a process described in Production Example 1 except that 2-methylcyclohexylamine was used in place of cyclohexylmethylamine.
The compound 2 of the present invention 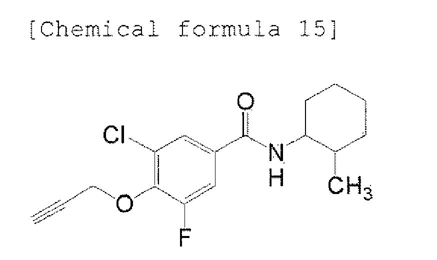 ¹H-NMR (CDCl₃) δ: 0.93 (0.8H, d, J = 6.8 Hz), 0.97 (2.2H, d, J = 6.3 Hz), 1.09-1.83 (8.0H, m), 1.93-2.07 (1.0H, m), 2.51-2.52 (1.0H, m), 3.63-3.72 (0.7H, m), 4.21-4.25 (0.3H, m), 4.88 (2.0H, t, J = 2.3 Hz), 5.72 (0.7H, d, J = 8.3 Hz), 5.98 (0.3H, d, J = 7.8 Hz), 7.44-7.49 (1.0H, m), 7.55-7.56 (1.0H, m).

### Production Example 3

N-(2-methylcyclopentyl)-3-chloro-5-fluoro-4-(2-propynyloxy)benzamide (hereinafter, referred to as a compound 3 of the present invention) was obtained according to a process described in Production Example 1, except that 2-methylcyclopentylamine was used in place of cyclohexylmethylamine.
The compound 3 of the present invention 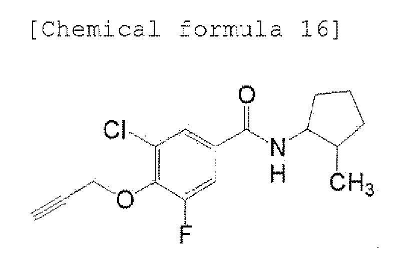 ¹H-NMR (CDCl₃ ) δ: 0.94 (2.0H d, J = 7.1 Hz), 1.08 (1.0H, d, J = 6.6 Hz), 1.25-2.30 (7.0H, m), 2.50-2.52 (1.0H, m), 3.90-3.97 (0.4H, m), 4.38-4.45 (0.6H, m), 4.88-4.91 (2.0H, m), 5.83-5.84 (1.0H, m), 7.44-7.49 (1.0H, m), 7.54-7.56 (1.0H, m).

### Production Example 4

N-(1-hydroxycyclohexyl)methyl-3-chloro-5-fluoro-4-(2-propynyloxy)benzamide (hereinafter, referred to as a compound 4 of the present invention) was obtained according to a process described in Production Example 1 except that 1-(1-hydroxycyclohexyl)methylamine hydrochloride was used in place of cyclohexylmethylamine.
The compound 4 of the present invention 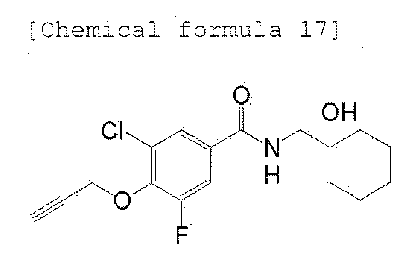 ¹H-NMR (CDCl₃) δ: 1.34-1.60 (10H, m), 2.03 (1H, s), 2.52 (1H, t, J = 2.4 Hz), 3.47 (2H, d, J = 5.8 Hz), 4.89 (2H, d, J = 2.4 Hz), 6.54-6.56 (1H, m), 7.51 (1H, dd, J = 11.0, 2.1 Hz), 7.62 (1H, dd, J = 2.1, 1.6 Hz).

### Production Example 5

N-(cyclohexylmethyl)-3-methoxy-5-methyl-4-(2-propynyloxy)benzamide (hereinafter, referred to as a compound 5 of the present invention) was obtained according to a process described in Production Example 1 except that 3-methoxy-5-methyl-4-(2-propynyloxy)benzoyl chloride was used in place of 3-chloro-5-fluoro-4-(2-propynyloxy)benzoyl chloride.
The compound 5 of the present invention 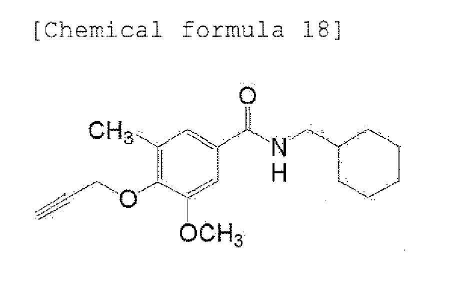 ¹H-NMR (CDCl₃ ) δ: 0.99-1.04 (2H, m), 1.16-1.27 (3H, m), 1.55-1.80 (6H, m), 2.35 (3H, s), 2.42 (1H, t, J = 2.4 Hz), 3.29 (2H, t, J = 6.5 Hz), 3.90 (3H, s), 4.76 (2H, d, J = 2.4 Hz), 6.08-6.10 (1H, m), 7.06 (1H, s), 7.29 (1H, s).

### Production Example 6

N-(2-methylcyclohexyl)-3-methoxy-5-methyl-4-(2-propynyloxy)benzamide (hereinafter, referred to as a compound 6 of the present invention) was obtained according to a process described in Production Example 1 except that 2-methylcyclohexylamine and 3-methoxy-5-methyl-4-(2-propynyloxy)benzoyl chloride were used in place of cyclohexylmethylamine and 3-chloro-5-fluoro-4-(2-propynyloxy)benzoyl chloride respectively.
The compound 6 of the present invention 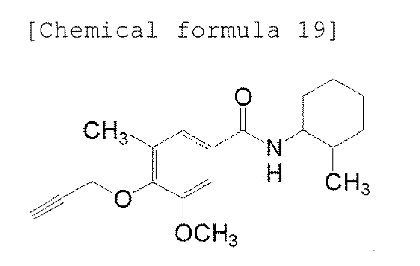 ¹H-NMR (CDCl₃) δ: 0.94 (1.0H, d, J = 7.1 Hz), 0.99 (2.0H, d, J = 6.6 Hz), 1.13-1.82 (8.0H, m), 1.93-2.07 (1.0H, m), 2.35 (2.0H, s), 2.37 (1.0H, s), 2.42 (1.0H, t, J = 2.4 Hz), 3.64-3.74 (0.7H, m), 3.90-3.90 (3.0H, m), 4.22-4.28 (0.3H, m), 4.76-4.77 (2.0H, m), 5.77 (0.7H, d, J = 9.3 Hz), 6.06 (0.3H, d, J = 8.8 Hz), 7.04-7.06 (1.0H, m), 7.29-7.30 (1.0H, m).

### Production Example 7

N-(1-hydroxycyclohexyl)methyl-3-methoxy-5-methyl-4-(2-propynyloxy)benzamide (hereinafter, referred to as a compound 7 of the present invention) was obtained according to a process described in Production Example 1 except that 1-(1-hydroxycyclohexyl)methylamine hydrochloride and 3-methoxy-5-methyl-4-(2-propynyloxy)benzoyl chloride were used in place of cyclohexylmethylamine and 3-chloro-5-fluoro-4-(2-propynyloxy)benzoyl chloride respectively.
The compound 7 of the present invention 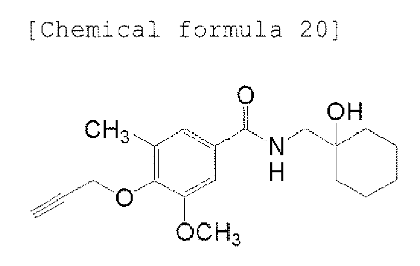 ¹H-NMR (CDCl₃) δ: 1.34-1.62 (10H, m), 1.77 (1H, s), 2.33 (3H, s), 2.43 (1H, t, J = 2.4 Hz), 3.47 (2H, d, J = 5.8 Hz), 3.88 (3H, s), 4.76 (2H, d, J = 2.4 Hz), 6.60-6.63 (1H, m), 7.14 (1H, s), 7.30 (1H, s).

### Production Example 8

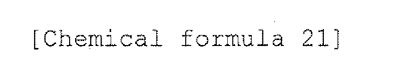

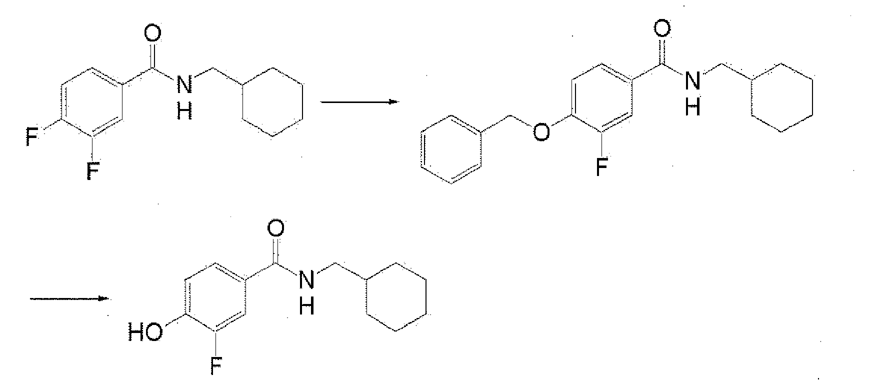

To a solution of 7.0 g of N-(cyalahexylmethyl)-3,4-difluorobenzamide and 3.6 g of benzyl alcohol in 70 ml of DMF was added 1.3 g of 60% sodium hydride (oily) at room temperature, and the mixture was stirred at 80°C for 4 hours. The reaction mixture was added to water. The resulting solid was collected by filtration, and washed with hexane, and a residue was obtained.
The residue was added to a mixture of 100 ml of ethyl acetate and 10 ml of methanol, and 100mg of 10% palladium carbon was added thereto. A reaction was performed under hydrogen atmosphere at a normal pressure for 6 hours. The reaction mixture was filtered with Celite. The filtrate was concentrated under reduced pressure, and the resulting residue was washed with MTBE, and 5.5 g of a product was obtained.

### N-(cyclohexylmethyl)-3-fluoro-4-hydroxybenzamide

¹H-NMR (CDCl₃) δ: 0.93-1.29 (5H, m), 1.52-1.80 (6H, m), 3.25 (2H, dd, J = 6.3, 6.3 Hz), 6.55-6.60 (1H, m), 6.98 (1H, t, J = 8.5 Hz), 7.43 (1H, dd, J = 8.4, 2.1 Hz), 7.56 (1H, dd, J = 11.8, 2.1 Hz), 9.41 (1H, s).

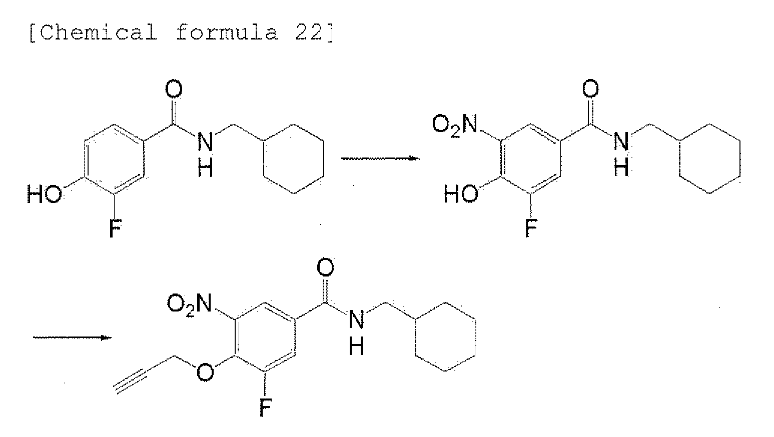

To 5 ml of concentrated sulfuric acid was added 0.50 g of N-(cyclohexylmethyl)-3-fluoro-4-hydroxybenzamide, and 0.20 g of 69% nitric acid was added thereto dropwise under ice-cooling. The mixture was stirred for 30 minutes. The reaction mixture was added to water. The resulting solid was collected by filtration, and dried, and a residue was obtained.
To a solution of the residue, 0.63 g of triphenylphosphine and 0.13 g of propargyl alcohol in 15 ml of THF was added dropwise 1.1 g of a solution (40%) of diethyl azodicarboxylate in toluene at room temperature. The mixture was stirred at room temperature for 4 hours. The reaction mixture was concentrated under reduced pressure, and the residue was subjected to silica gel column chromatography, and 0.40 g of N-(cyclohexylmethyl)-3-fluoro-5-nitro-4-(2-propynyloxy)benzamide (hereinafter, referred to as a compound 8 of the present invention) was obtained.
The compound 8 of the present invention 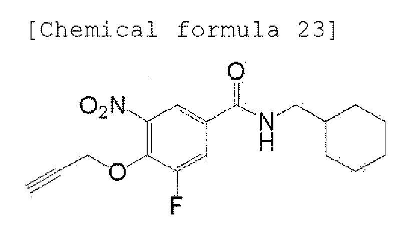 ¹H-NMR (CDCl₃) δ: 0.95-1.30 (5H, m), 1.54-1.79 (6H, m), 2.56 (1H, t, J = 2.4 Hz), 3.31 (2H, dd, J = 6.5, 6.5 Hz), 4.97 (2H, d, J = 2.4 Hz), 6.21-6.26 (1H, m), 7.86 (1H, dd, J = 11.1, 2.1 Hz), 7.98 (1H, dd, J = 1.7, 2.1 Hz).

The following Reference Production Examples show production of intermediates for producing the compound of the present invention.

### Reference Production Example 1

To 80 ml of DMF were added 7.9 g of 3-chloro-4,5-difluorobenzoic acid, 5.9 g of propargyl bromide and 6.8 g of potassium carbonate, and the mixture was stirred at room temperature for 12 hours and then at 80°C for 1 hour. The reaction mixture was allowed to cool to near room temperature, and ethyl acetate was added thereto, followed by filtration through Celite. To the filtrate were added sequentially water and diluted hydrochloric acid, followed by extraction with ethyl acetate. The organic layer was dried over magnesium sulfate, and then concentrated under reduced pressure, and 9.0 g of 2-propynyl 3-chloro-4,5-difluorobenzoate was obtained.

### 2-Propynyl 3-chloro-4,5-difluorobenzoate

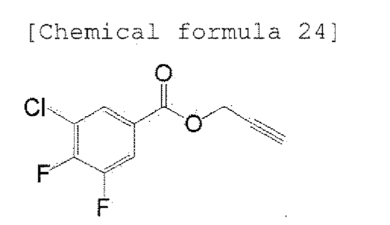

¹H-NMR (CDCl₃ ) δ: 2.56 (1H, t, J = 2.6 Hz), 4.93 (2H, d, J = 2.4 Hz), 7.81 (1H, ddd, J = 9.9, 6.8, 2.1 Hz), 7.94 (1H, ddd, J = 6.0, 2.1, 1.9 Hz).

To a solution of 9.0 g of 2-propynyl 3-chloro-4,5-difluorobenzoate and 2.9 g of propargyl alcohol in 50 ml of DMF was added 1.7 g of 60% sodium hydride (oily) at 0°C, and the mixture was stirred at 0°C for 30 minutes and then at room temperature for 1 hour. To the reaction mixture was added water, followed by extraction with ethyl acetate. The organic layer was dried over magnesium sulfate, and then concentrated under reduced pressure. The residue was subjected to silica gel column chromatography, and 5.3 g of 2-propynyl 3-chloro-5-fluoro-4-(2-propynyloxy)benzoate was obtained.

### 2-Propynyl 3-chloro-5-fluoro-4-(2-propynyloxy)benzoate

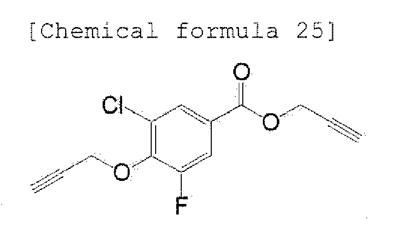

¹H-NMR (CDCl₃) δ: 2.53 (1H, t, J = 2.4 Hz), 2.54 (1H, t, J = 2.4 Hz), 4.91-4.93 (4H, m), 7.74 (1H, dd, J = 11.1, 2.0 Hz), 7.92 (1H, dd, J = 2.0, 1.8 Hz).

To a mixed solution of 20 ml of ethanol, 10 ml of tetrahydrofuran, 10 ml of water and 1.8 g of sodium hydroxide was added 5.0 g of 2-propynyl 3-chloro-5-fluoro-4-(2-propynyloxy)benzoate, followed by stirring at room temperature for 2 hours. The reaction mixture was concentrated under reduced pressure. The residue was made acidic by addition of hydrochloric acid. Then, a precipitated solid was collected by filtration, and 4.5 g of 3-chlorc-5-fluorc-4-(2-propynyloxy)benzoic acid was obtained.

### 3-Chloro-5-fluoro-4-(2-propynyloxy)benzoic acid

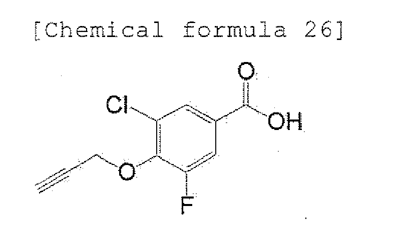

¹H-NMR (CDCl₃) δ: 2.54 (1H, t, J = 2.4 Hz), 4.94 (2H, d, J = 2.4 Hz), 7.77 (1H, dd, J = 11.0, 1.8 Hz), 7.96 (1H, dd, J = 1.9, 1.8 Hz).

To 20 ml of toluene were added 3.0 g of 3-chloro-5-fluoro-4-(2-propynyloxy)benzoic acid, 1.0 ml of thionyl chloride and 10 mg of DMF, followed by heating to reflux for 4 hours. The reaction mixture was allowed to cool to near room temperature and then concentrated under reduced pressure, and 2.4 g of 3-chloro-5-fluoro-4-(2-propynyloxy)benzoyl chloride was obtained. 3-Chloro-5-fluoro-4-(2-propynyloxy)benzoyl chloride 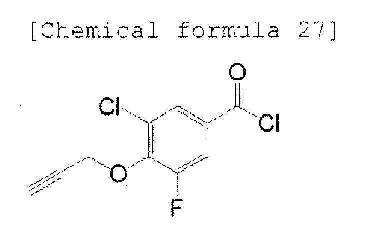 ¹H-NMR (CDCl₃) δ: 2.55 (1H, t, J = 2.4 Hz), 4.99-5.00 (2H, m), 7.81 (1H, dd, J = 11.2, 2.2 Hz), 8.00 (1H, dd, J = 2.2, 1.4 Hz).

### Reference Production Example 2

To 50 ml of DMF were added 4.4 g of 4-hydroxy-3-methoxy-5-methylbenzoic acid, 6.3 g of propargyl bromide and 7.3 g of potassium carbonate, and the mixture was stirred at room temperature for 1 hour and then at 80°C for 1 hour. To the reaction mixture was added ethyl acetate, followed by filtration through Celite. To the filtrate were added sequentially water and diluted hydrochloric acid, followed by extraction with ethyl acetate. The organic layer was dried over magnesium sulfate, and then concentrated under reduced pressure. The residue was subjected to silica gel column chromatography, and 3.2 g of 2-propynyl 3-methoxy-5-m-ethyl-4-(2-propynyloxy)benzoate was obtained.

### 2-Propynyl 3-methoxy-5-methyl-4-(2-propynyloxy)benzoate

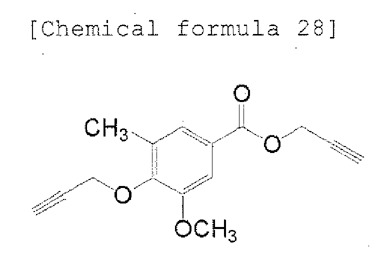

¹H-NMR (CDCl₃) δ: 2.35 (3H, s), 2.43 (1H, t, J = 2.4 Hz), 2.51 (1H, t, J = 2.4 Hz), 3.90 (3H, s), 4.80 (2H, d, J = 2.4 Hz), 4.90 (2H, d, J = 2.4 Hz), 7.46 (1H, d, J = 2.0 Hz), 7.56 (1H, dd, J = 2.0, 0.7 Hz).

To a mixed solution of 20 ml of ethanol, 10 ml of tetrahydrofuran, 10ml of water and 1.2 g of sodium hydroxide was added 5.0 g of 2-propynyl 3-methoxy-5-methyl-4-(2-propynyloxy)benzoate, and the mixture was stirred at room temperature for 2 hours. The reaction mixture was concentrated under reduced pressure. The residue was made acidic by addition of hydrochloric acid. Then, a precipitated solid was collected by filtration, and 2.2 g of 3-methoxy-5-methyl-4-(2-propynyloxy)benzoic acid was obtained.

### 3-Methoxy-5-methyl-4-(2-propynyloxy)benzoic acid

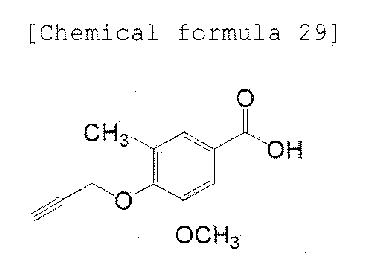

¹H-NMR (CDCl₃) δ: 2.37 (3H, s), 2.44 (1H, t, J = 2.3 Hz), 3.92 (3H, s), 4.82 (2H, d, J = 2.2 Hz), 7.50 (1H, s), 7.61 (1H, s).

To 20 ml of toluene were added 1.1 g of 3-methoxy-5-methyl-4-(2-propynyloxy)benzoic acid, 0.5 ml of thionyl chloride and 10 mg of DMF, and the mixture was heated to reflux for 4 hours. The reaction mixture was concentrated under reduced pressure, and 1.0 g of 3-methoxy-5-methyl-4-(2-propynyloxy)benzoyl chloride was obtained. 3-Methoxy-5-methyl-4-(2-propynyloxy)benzoyl chloride 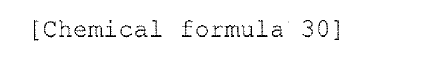 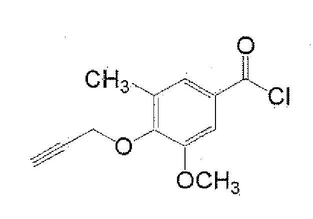 ¹H-NMR (CDCl₃) δ: 2.37 (3H, s), 2.45-2.45 (1H, m), 3.92 (3H, s), 4.86 (2H, d, J = 2.4 Hz), 7.50-7.50 (1H, m), 7.66-7.67 (1H, m).

Then, Formulation Examples are shown. The term "part(s)" represents part(s) by weight.

### Formulation Example 1

50 parts of each of the compounds 1 to 8 of the present invention, 3 parts of calcium ligninsulfonate, 2 parts of magnesium laurylsulfate and 45 parts of synthetic hydrated silica are pulverized and mixed well to give a wettable powder of each compound.

### Formulation Example 2

20 parts of each of the compounds 1 to 8 of the present invention and 1.5 parts of sorbitan trioleate are mixed with 28.5 parts of an aqueous solution containing 2 parts of polyvinyl alcohol, and wet-pulverized finely. To the obtained mixture, 40 parts of an aqueous solution containing 0.05 part of xanthan gum and 0.1 part of aluminium magnesium silicate is added and further 10 parts of propylene glycol is added. The mixture was stirred and mixed to give a flowable of each compound.

### Formulation Example 3

2 parts of each of the compounds 1 to 8 of the present invention, 88 parts of kaolin clay and 10 parts of talc are pulverized and mixed well to give a dust of each compound.

### Formulation Example 4

5 parts of each of the compounds 1 to 8 of the present invention, 14 parts of polyoxyethylenestyryl phenyl ether, 6 parts of calcium dodecylbenzenesulfonate and 75 parts of xylene are mixed well to give an emulsifiable concentrate of each compound.

### Formulation Example 5

2 parts of each of the compounds 1 to 8 of the present invention, 1 part of synthetic hydrated silica, 2 parts of calcium ligninsulfonate, 30 parts of bentonite and 65 parts of kaolin clay are pulverized and mixed well, and water is added thereto and kneeded well, granulated and dried to give granules of each compound.

### Formulation Example 6

10 parts of each of the compounds 1 to 8 of the present invention, 35 parts of white carbon containing 50 parts of ammonium polyoxyethylenealkyl ether sulfate, and 55 parts of water are mixed and wet pulverized finely to give a formulation of each compound.

The following Test Examples show that the compounds of the present invention are useful for controlling a plant disease.
The controlling effect was evaluated by visually observing the area or a lesion spots on each of test plants at the time of investigation and comparing the area of lesion spots on a plant treated with the compound of the present invention with that on an untreated plant.

### Test Example 1

### Test of preventive effect on wheat powdery mildew (Erysiphe graminis f. sp. tritici)

Each of plastic pots was filled with sandy loam and sown with wheat (cultivar; Shiragane), followed by growing in a greenhouse for 10 days. Each of the compounds 2 and 3 of the present invention was formulated into a flowable formulation according to Formulation Example 6. The flowable formulation was diluted to a predetermined concentration (500 ppm) with water, and foliage application of the dilution was carried out so that the dilution adhered sufficiently to the surfaces of leaves of the grown wheat young seedling. After the foliage application, the plant was air-dried and then inoculated by sprinkling with spores of *Erysipne graminis f. sp.* tritici. After the inoculation, the plant was held in a greenhouse at 23°C for 7 days and the area of lesion spots was investigated. As a result, it was found that the area of lesion spots on the plant treated with each of the compounds 2 and 3 of the present invention was 30% or less of that on an untreated plant.

### Test Example 2

### Test of preventive effect on wheat Glume blotch (Stagonospora nodorum)

Each of plastic pots was filled with sandy loam and sown with wheat (cultivar; Shirogane), followed by growing in a greenhouse for 10 days. Each of the compounds 2 and 3 of the present invention was formulated into a flowable formulation according to Formulation Example 6. The flowable formulation was diluted to a predetermined concentration (500 ppm) with water, and foliage application of the dilution was carried out so that the dilution adhered sufficiently to the surfaces of leaves of the grown wheat young seedling. After the foliage application, the plant was air-dried and then inoculated by spraying a water suspension of spores of *Stagonospora nodorum.* After the inoculation, the plant was held under darkness and high humidity conditions at 18°C for 4 days and held under lighting conditions for 4 days, and thenthe area of lesion spots was investigated. As a result, it was found that the area of lesion spots on the plant treated with each of the compounds 2 or 3 of the present invention was 30% or less of that on an untreated plant.

### Test Example 3

### Test of preventive effect on wheat Fusarium blight (Fusarium culmorum)

Each of plastic pots was filled with sandy loam and sown with wheat (cultivar; Shirogane), followed by growing in a greenhouse for 10 days. The compound 1 of the present invention was formulated into a flowable formulation according to Formulation Example 6. The flowable formulation was diluted to a predetermined concentration (500 ppm) with water, and foliage application of the dilution was carried out so that the dilution adhered sufficiently to the surfaces of leaves of the grown wheat young seedling. After the foliage application, the plant was air-dried and then inoculated by spraying a water suspension of spores of *Fusarium culmorum.* After the inoculation, the plant was held under darkness and high humidity conditions at 23°C for 4 days and held under lighting conditions for 3 days, and then the area of lesion spots was investigated. As a result, it was found that the area of lesion spots on the plant treated with the compound 1 of the present invention was 30% or less of that on an untreated plant.

### Test Example 4

### Test of preventive effect on cucumber stem rot (Sclerotinia sclerotiorum)

Each of plastic pots was filled with sandy loam and sown with cucumber (variety; Sagamihanjiro), followed by growing in a greenhouse for 12 days. The compound 2 of the present invention was formulated into a flowable formulation according to Formulation Example 6. The flowable formulation was diluted to a predetermined concentration (500 ppm) with water, and foliage application of the dilution was carried out so that the dilution adhered sufficiently to the surfaces of leaves of the grown cucumber young seedling. After the foliage application, the plant was air-dried and a PDA medium containing mycelia of Sclerotinia sclerotiorum was placed on the surfaces of the cucumber leaves. After the inoculation, the plant was grown at 18°C and high humidity for 4 days. Then, the area of lesion spots was investigated. As a result, it was found that the area of lesion spots on the plant treated with the compound 2 of the present invention was 30% or less of that on an untreated plant.

### Test Example 5

### Test of preventive effect on tomato late blight (Phytophthora infestans)

Each of plastic pots was filled with sandy loam and sown with tomato (cultivar; Patio), followed by growing in a greenhouse for 20 days. Each of the compounds 3, 4 and 7 of the present invention was formulated into a flowable formulation according to Formulation Example 6. The flowable formulation was diluted to a predetermined concentration (500 ppm) with water, and foliage application of the dilution was carried out so that the dilution adhered sufficiently to the surfaces of leaves of the grown tomato young seedling. After the plant was air-dried so that the diluted solution on leaves was dried, a water suspension of zoosporangia of *Phytophthora infestans* was sprayed. After the inoculation, the plant was held under high humidity conditions at 23°C for 1 day and held in a greenhouse for 4 days, and then the area of lesion spots was investigated. As a result, it was found that the area of lesion spots on the plant treated with each of the compounds 3, 4 and 7 of the present invention was 30% or less of that an untreated plant.

### Industrial Applicability

As described above, according to the present invention, an amide compound having excellent plant disease controlling activity which is useful as an active ingredient of a plant disease controlling agent can be provided.

## Claims

1. An amide compound represented by the formula (1): 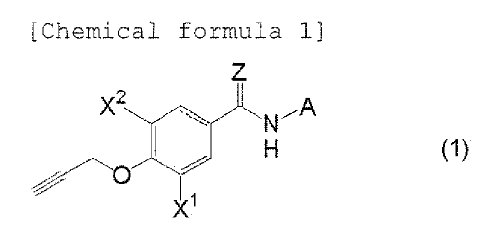 wherein,
X¹ represents a fluorine atom or a methoxy group,
X² represents a chlorine atom, a bromine atom, an iodine atom, a C₁-C₄ alkyl group, a C₂-C₄ alkenyl group, a C₂-C₄ alkynyl group, a C₁-C₄ haloalkyl group, a C₂-C₄ alkoxy group, a C₁-C₄ alkylthio group, a hydroxy C₁-C₄ alkly group, a nitro group, a cyano group, a formyl group, a NR¹R² group, a CO₂R³ group, a CONR⁴R⁵ group, or a phenyl group optionally substituted with at least one group selected from the group consisting of a methyl group, a halogen atom, a cyano group and a nitro group,
Z represents an oxygen atom or a sulfur atom,
A represents an A¹-CR¹¹R¹²R¹³ group or A²-Cy group,
A¹ represents a single bond or a CH₂ group,
A² represents a single bond, a CH₂ group, a CH(CH₃) group, a C(CH₃)₂ group or a CH(CH₂CH₃) group,
Cy represents a C₃-C₆ cycloalkyl group optionally substituted with at least one group selected from the group consisting of a C₁-C₄ alkyl group, a C₂-C₄ alkenyl group, a C₂-C₄ alkynyl group, a halogen atom, a hydroxyl group, a cyano group, a carboxyl and a C₂-C₅ alkoxycarbonyl group,
R¹ and R² independently represent a hydrogen atom, a C₁-C₄ alkyl group, a C₃-C₄ alkenyl group, a C₃-C₄ alkynyl group, a C₂-C₄ haloalkyl group, a C₂-C₅ alkylcarbonyl group, a C₂-C₅ alkoxycarbonyl group or a C₁-C₄ alkylsulfonyl group,
R³ represents a C₁-C₄ alkyl group, a C₃-C₄ alkenyl group or a C₃-C₄ alkynyl group,
R⁴ represents a hydrogen atom, a C₁-C₄ alkyl group, a C₃-C₄ alkenyl group, a C₃-C₄ alkynyl group, a C₂-C₄ haloalkyl group, a C₂-C₅ alkylcarbonyl group, a C₂-C₅ alkoxycarbonyl group, or a C₁-C₄ alkylsulfonyl group,
R⁵ represents a hydrogen atom, a C₁-C₄ alkyl group, a C₃-C₄ alkenyl group, a C₃-C₄ alkynyl group or a C₂-C₄ haloalkyl group,
R¹¹ and R¹² independently represent a C₁-C₄ alkyl group, and
R¹³ represents a hydrogen atom, a C₁-C₄ alkyl group, a C₂-C₄ alkenyl group, a C₂-C₄ alkynyl group, a cyano group, a carboxyl group or a C₂-C₅ alkoxycarbonyl group.

2. The amide compound according to claim 1, wherein X¹ is a fluorine atom in the formula (1).

3. The amide compound according to claim 1, wherein X¹ is a methoxy group in the formula (1).

4. The amide compound according to claim 1, wherein A is A¹-CR¹¹R¹²R¹³ and R¹³ is a hydrogen atom or a methyl group in the formula (1).

5. The amide compound according to claim 1, wherein A is A²-Cy and Cy is a C₃-C₆ cycloalkyl group optionally substituted with at least one group selected from the group consisting of a C₁-C₄ alkyl group, a halogen atom, a hydroxyl group and a cyano group in the formula (1).

6. A plant disease controlling agent comprising the amide compound according to any one of claims 1 to 5 as an active ingredient.

7. A method of controlling a plant disease which comprises treating a plant or soil with an effective amount of the amide compound according to any one of claims 1 to 5.
